(19) Europäisches Patentamt
European Patent Office
Office européen des brevets

(11) Veröffentlichungsnummer: **0 167 973**
**B1**

# (12) EUROPÄISCHE PATENTSCHRIFT

(45) Veröffentlichungstag der Patentschrift: **22.06.88**

(51) Int. Cl.⁴: **C 07 D 263/32**, C 07 D 277/22, G 01 N 31/22

(21) Anmeldenummer: **85108203.2**

(22) Anmeldetag: **03.07.85**

(54) **Redox-Indikatoren.**

(30) Priorität: **07.07.84 DE 3425118**

(43) Veröffentlichungstag der Anmeldung: **15.01.86 Patentblatt 86/3**

(45) Bekanntmachung des Hinweises auf die Patenterteilung: **22.06.88 Patentblatt 88/25**

(84) Benannte Vertragsstaaten: **AT BE CH DE FR GB IT LI LU NL SE**

(56) Entgegenhaltungen:
**EP - A - 0 010 652**
**AT - B - 209 341**
**AT - B - 322 551**
**DE - A - 2 735 690**
**DE - A - 2 803 955**
**DE - A - 3 026 054**

(73) Patentinhaber: **BOEHRINGER MANNHEIM GMBH, Sandhofer Strasse 116, D-6800 Mannheim 31 (DE)**

(72) Erfinder: **Deneke, Ulfert, Dr. rer. nat., Birkenweg 5, D-6149 Rimbach-Zotzenbach (DE)**
Erfinder: **Güthlein, Werner, Dr. rer. nat., Im Sennteich 31, D-6800 Mannheim, 24 (DE)**
Erfinder: **Weckerle, Wolfgang, Dr. rer. nat., Auf dem Leimen 12, D-6718 Grünstadt (DE)**
Erfinder: **Wielinger, Hans, Dr. phil., Im Langgewann 7, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Reaktion von Wasserstoffperoxid mit Oxidationsindikatoren unter Katalyse von Peroxidase oder peroxidatisch wirksamen Substanzen spielt in der analytischen Chemie eine besondere Rolle, weil sie ausser dem Nachweis von Wasserstoffperoxid und Peroxidase auch die Bestimmung einer Reihe von Stoffen gestattet, die mit Sauerstoff und einer Reihe von Stoffen unter Bildung von Wasserstoffperoxid reagieren. Im folgenden seien einige dieser Stoffe als Beispiele aufgezählt und in Klammern die entsprechenden Oxidasen erwähnt:

Glucose (Glucoseoxidase), Galactose (Galaxtoseoxidase), L-Aminosäuren (L-Aminosäurenoxidase), Cholesterin (Cholesterinoxidase), Harnsäure (Uricase), Sarkosin (Sarkosinoxidase), Glycerin (Glycerinoxidase), Pyruvat (Pyruvatoxidase).

Als Nachweisreaktion für Peroxidasen kommt die Methode insbesondere für die Bestimmung von Hämoglobin infrage.

Es sind dies vor allem Reaktionen, die in der medizinischen Diagnostik und in der Lebensmittelchemie von grosser Bedeutung sind.

Die Nachweisreaktionen werden entweder in der Küvette oder mit Hilfe von Trockenreagenzträgern durchgeführt. Die Quantifizierung erfolgt dabei mit Photometern über eine Transmissionsmessung, mit Remissionsphotometern über Remissionsmessung oder mit Hilfe von Vergleichsfarben durch visuellen Vergleich.

Der Einsatz von Trockenreagenzträgern, d.h. saugfähigen oder quellfähigen Trägern, die mit den Reagenzien imprägniert sind bzw. in die die Reagenzien über andere Schritte eingearbeitet sind und auf denen nach Befeuchten mit dem Substrat die Nachweisreaktion abläuft, hat in jüngster Zeit immer mehr an Bedeutung zugenommen. Diese Hilfsmittel ermöglichen durch eine einfache Handhabung, bei gleichzeitig grosser Zeitersparnis eine entscheidende Rationalisierung der entsprechenden Analysen. Die Forderung Trockenreagenzien zu entwickeln, bei denen mit unverdünnten Proben gearbeitet werden kann, stellt den Entwickler hinsichtlich der Auswahl des einzusetzenden Indikators bzw. Indikatorsystems vor das Problem, dass das Serum oder Plasma (im folgenden als Serum bezeichnet) die Nachweisreaktionen stark stört. Diese Störungen machen sich vor allem dann bemerkbar, wenn es notwendig ist, die Substrate bzw. Enzymaktivitäten über gekoppelte Reaktionsschritte nachzuweisen. Hier seien als Beispiel für Substrate die Nachweise von Creatinin und Harnsäure sowie als Beispiel für die Aktivitätsbestimmungen von Enzymen die Bestimmungen von Creatinkinase, Glutamat-Oxalacetat-Transaminase (GOT) und Glutamat-Pyruvat-Transaminase (GPT) erwähnt.

In der Literatur sind zahlreiche Verbindungen bekannt, die als Indikatoren für den Nachweis von Wasserstoffperoxid mit Peroxidase als Katalysator eingesetzt werden können. Solche Indikatoren sind: Benzidin und Benzidin-Derivate, verschiedene Phenole, Polyphenole wie z.B. Guajakharz, Leukofarbstoffe wie z.B. Leukomalachitgrün, Dichlorphenolindophenol, Aminocarbazole, Triarylimidazole, 2,2′-Azino-di-[3-ethyl-benzthiazol-sulfonsäure-(6)] sowie Farbstoffe, die als Kupplungsprodukt der oxidativen Kupplung von Aminoantipyrin oder verwandten Stoffen mit Phenolen, Naphtholen, Anilinderivaten und anderen Kupplungskomponenten entstehen.

Beim Nachweis von $H_2O_2$ in unverdünnten Serumproben weisen die vorstehenden bekannten Indikatoren mehr oder weniger starke Störungen durch Reaktion mit anderen Serumbestandteilen auf, die eine höhere oder meistens geringere Konzentration des nachzuweisenden Substrates vortäuschen. Relativ wenig gestört werden einige Triarylimidazole, wie sie in der DE-OS 2 735 690 beschrieben sind. Die beschriebenen Imidazole sind nur im saurem pH-Bereich stabil und werden, wie Experimente zeigten, beim Überführen in einen schwachsauren bis schwach alkalischen pH-Bereich, wie er bei fast allen enzymatischen Reaktionen notwendig ist, also dann, wenn sie als freie Base vorliegen, spontan vom Luftsauerstoff oxidiert. Eine Verarbeitung zu funktionsfähigen Trockenreagenzien mit diesen Indikatoren ist deshalb nur möglich, wenn sie in ein Schutzkolloid, wie z.B. Gelatine, eingebettet werden. Dies ist jedoch nur in speziellen Fällen durchführbar.

Die Aufgabenstellung der vorliegenden Erfindung war es daher, Farbstoffbildner für die Nachweisreaktion von Wasserstoffperoxid bzw. peroxidatisch wirksame Substanzen zu finden, die mit den im Serum enthaltenen Störsubstanzen nicht reagieren, im schwachsauren bis alkalischen Bereich von Luftsauerstoff nicht spontan oxidiert werden und somit sowohl in Küvettentests als auch in allen für Trockenreagenzträger brauchbaren Matrices verwendbar sind.

Es wurde überraschenderweise gefunden, dass Verbindungen der allgemeinen Formel I den oben gestellten Anforderungen entsprechen.

Gegenstand der Erfindung sind daher Oxazol- bzw. Thiazolderivate der Formel I

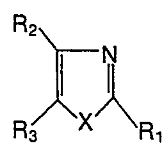

(I),

in der

X Sauerstoff oder Schwefel ist,

$R_1$ Julolidin oder Tetrahydrochinolin, das am Stickstoffatom eine Alkylgruppe mit 1–6 Kohlenstoffatomen tragen kann, die wiederum durch einen Sulfonsäure-, Phosphonsäure- oder Carbonsäurerest substituiert sein kann, oder die Gruppe

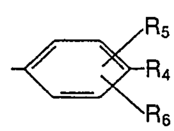

,

in der

$R_4$ eine Hydroxy-, eine mono- oder dialkylierte Aminogruppe, wobei die Alkylgruppen 1–6 Kohlenstoffatome aufweisen und ein- oder mehrfach durch Halogen, Hydroxy, Methoxy, Carboxyl, einen Sulfonsäure- oder Phosphonsäurerest, der durch Methanol oder Ethanol verestert sein kann, oder einen Morpholinorest substituiert sein können,

$R_5$ und $R_6$, die gleich oder verschieden sein können, Wasserstoff, $C_1$–$C_6$-Alkyl, das ein- oder mehrfach durch Halogen, Hydroxy, Methoxy, Carboxyl, einen Sulfonsäure- oder Phosphonsäurerest, der durch Methanol oder Ethanol verestert sein kann, oder einen Morpholinorest substituiert sein kann, oder $C_1$–$C_6$-Alkoxy, das durch eine Carboxylgruppe substituiert sein kann, bedeuten,

$R_2$ Wasserstoff, einen Alkylrest mit 1–6 Kohlenstoffatomen, der ein- oder mehrfach durch Halogen, Hydroxy, Methoxy, Carboxyl, einen Sulfonsäure- oder Phosphonsäure-Rest, der durch Methanol oder Ethanol verestert sein kann, oder einen Morpholinorest substituiert sein kann, sowie Julolidin, Tetrahydrochinolin, das am Stickstoffatom eine Alkylgruppe mit 1–6 Kohlenstoffatomen tragen kann, die durch einen Carboxyl-, Phosphonsäure- oder Sulfonsäurerest substituiert sein kann, oder die Gruppe

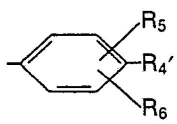

in der

$R_5$ und $R_6$ die bei $R_1$ angegebene Bedeutung haben und

$R_4'$ eine Hydroxy-, eine Amino- oder eine mono- oder dialkylierte Aminogruppe darstellt, wobei die Alkylgruppen 1–6 Kohlenstoffatome aufweisen und ein- oder mehrfach durch Halogen, Hydroxy, $C_1$–$C_6$-Alkoxy, Carboxyl, einen Sulfonsäure- oder Phosphonsäurerest, der durch Methanol oder Ethanol verestert sein kann, oder einen Morpholinorest substituiert sein können,

bedeuten und

$R_3$ die gleiche Bedeutung wie $R_2$ hat oder Cycloalkyl mit 3–7 Kohlenstoffatomen, Phenyl, Pyridyl oder einen durch Di-$C_1$–$C_6$-alkylamino oder Phenyl substituierten $C_1$–$C_6$-Alkylrest darstellt,

sowie deren Salze.

Der Ausdruck Alkyl in den Substituenten $R_2$, $R_3$, $R_4$, $R_4'$, $R_5$ und $R_6$ bedeutet Reste mit 1–6, vorzugsweise 1–4 Kohlenstoffatomen. Bevorzugt sind die Methyl-, Ethyl-, Propyl-, Butyl- und tert.Butyl-Gruppen. Die Aminogruppen der Phenylsubstituenten sind in der Regel durch Alkylgruppen mit 1–4 Kohlenstoffatomen, insbesondere durch Methyl substituiert. Sämtliche Alkyl-Gruppen können ihrerseits wiederum ein- oder mehrfach durch Halogen, Hydroxy, Methoxy, Carboxyl, einen Sulfonsäure- oder Phosphonsäure-Rest, der auch durch Methanol oder Ethanol verestert sein kann, und einen Morpholino-Rest substituiert sein.

Der Tetrahydrochinolin-Rest ist ein 1,2,3,4-Tetrahydrochinolin-Rest und kann am Stickstoff eine Alkylgruppe mit 1–6 Kohlenstoffatomen, vorzugsweise Methyl, tragen. Diese Alkylgruppe kann wiederum einen Carboxyl-, Phosphonsäure- oder Sulfonsäure-Rest tragen.

Cycloalkylgruppen der Substituenten $R_3$ sind Gruppen mit 3–7 Kohlenstoffatomen, insbesondere der Cyclopropyl-, Cyclopentyl- und Cyclohexyl-Rest.

Unter Alkoxy der Substituenten $R_2$, $R_4'$, $R_5$ und $R_6$, alleine oder als Substituenten von Alkylgruppen, sind Gruppen mit 1–6, vorzugsweise 1–4 Kohlenstoffatomen zu verstehen. Bevorzugt sind der Methoxy-, Ethoxy- und Propoxy-Rest.

Bevorzugte Substituenten $R_1$, $R_2$ oder $R_3$ sind 3,5-Dimethoxy-4-hydroxyphenyl-, 3,5-Di-tert.butyl-4-hydroxyphenyl-, 4-Dimethylaminophenyl-, 9-Julolidino- sowie 6-N-Methyl-1,2,3,4-tetrahydrochinolino, wobei die Alkylsubstituenten Carboxyl-, Phosphonsäure- oder Sulfonsäure-Gruppen tragen können. $R_2$ und $R_3$ können ausserdem bevorzugt Methyl bedeuten. $R_3$ bedeutet insbesondere ausser den vorstehend genannten Resten Wasserstoff, n-Butyl, tert.Butyl, 3-Methoxypropyl, 4-Methoxybutyl, 5-Methoxypentyl, Cyclohexyl, Phenyl, Benzyl, Hydroxymethyl, Pyridyl und Dimethylaminomethyl.

Die Sulfonsäure-, Phosphonsäure- und Carbonsäure-Reste, mit denen vorwiegend Alkyl-Gruppen substituiert sind, dienen insbesondere zur Löslichkeitsverbesserung.

Die Indikatoren gemäss Formel I lassen sich in alle bekannten Nachweissysteme einarbeiten.

Es lassen sich mit den Indikatoren Tests herstellen, die in der Küvette vermessen werden. Dazu wird der Indikator zusammen mit Peroxidase, dem/den für den jeweiligen Parameternachweis notwendigen Enzym/en, anderen Reagenzien, dem Puffersystem, gegebenenfalls Netzmittel und anderen Hilfsstoffen lyophilisiert, als Pulver vermischt oder zu Tabletten gepresst. Die so gewonnene Reagenzmischung wird vor Gebrauch in Wasser gelöst und so die Reagenzlösung bereitet. Nach Zugabe der Probe (Substratlösung, Enzymlösung, Serum oder Plasma) wird die entstandene Farbe am Photometer vermessen und über den molaren Extinktionskoeffizienten und die zugesetzten Reagenz- bzw. Probevolumina die jeweilige Konzentration bzw. Enzymaktivität berechnet. Es sind sowohl kinetische als auch Endpunkts-Messungen möglich.

Ebenso können die Indikatoren zusammen mit Peroxidase, dem/den für den jeweiligen Parameternachweis notwendigen Reagenzien bzw. anderen Enzymen, dem Puffersystem, gegebenenfalls Netzmitteln und anderen Hilfsstoffen auf saugfähigen Reagenzträgern wie Papiere, Vliese etc. imprägniert werden. Dazu kann man eine oder mehrere Imprägnierlösungen in Form von wässrigen oder organischen oder gemischten Lösungen herstellen, je nachdem wie sich die Reagenzien oder Hilfsstoffe lösen. Mit diesen Lösungen werden Träger imprägniert oder besprüht. Anschliessend wird getrocknet. Die so gewonnenen Reagenzträger können entweder als Schnelldiagnostika zur direkten Bestimmungen von Inhaltsstoffen aus z.B. Körperflüssigkeiten eingesetzt werden. Dabei wird die Körperflüssigkeit direkt auf den Reagenzträger gebracht oder dieser in diese eingetaucht. Durch Vergleichen der entstandenen Farbe mit Vergleichsfarben ist eine semiquantitative Bestimmung möglich. Über remissionsphotometrische Verfahren kann quantitativ ausgewertet werden. Es kann auch durch Eluieren der, wie vorstehend beschrieben, auf ein Papier oder Vlies imprägnierten Reagenzien mit Wasser oder Puffer eine Reagenzlösung hergestellt werden, mit der man wie oben beschrieben, Substrate oder Enzyme in einer Küvette am Photometer bestimmt (vgl. DE-OS 2 301 999).

Eine weitere Möglichkeit der Verwendung der erfindungsgemässen Indikatoren ist deren Einsatz in Reagenzfilmen zur quantitativen Bestimmung von Enzymen oder Substraten mittels eines Remissionsphotometers. Dabei wird der Indikator gemeinsam mit den anderen notwendigen Reagenzien und Hilfsstoffen, z.B. gemäss Verfahren entsprechend DE-PS 1 598 153 oder DE-OS 2 910 134 zu Reagenzfilmen verarbeitet.

Weiter hat sich gezeigt, dass die erfindungsgemässen Substanzen auch mit Stabilisatoren, wie sie in DE-PS 2 716 060 beschrieben sind, erfolgreich kombiniert werden können. Diese Stabilisatoren (1-Aryl-Semicarbazide) führen dazu, dass fertige Tests gegen Lichteinfluss unempfindlich werden und dass mit grösseren Mengen die Funktionskurven der remissionsphotometrischen Messungen moduliert werden können.

Die erfindungsgemässen Indikatoren können, wie gesagt, in allen üblichen Reagenzträgern, d.h. saugfähige Träger wie Filterpapier, Vliesen usw. oder quell- bzw. saugfähige Reagenzfilme eingearbeitet werden (DE-PS 1 598 153, DE-OS 2 910 134, DE-OS 3 247 608).

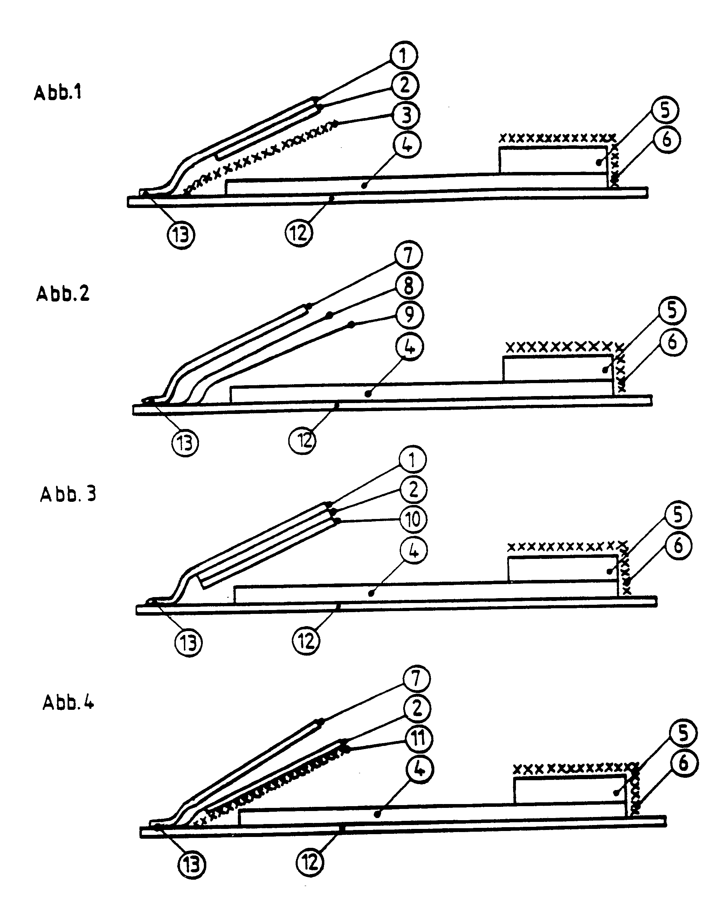

Da sie vorzugsweise jedoch zum Nachweis von Enzymen und Substraten in Serum Verwendung finden sollen, sind in den Abbildungen 1–4 im Querschnitt eine Reihe von Vorrichtungen dargestellt, die gemäss in DE-OS 3 029 579 einerseits aus Vollblut das für den Test erforderliche Serum bzw. Plasma abtrennen und andererseits aufgrund eines speziell gestalteten Aufbaus der Reagenz- und Hilfsstoffschichten eine Temperierung, Vorreaktion und gezieltes Starten der Hauptreaktion erlauben.

Im einzelnen sind die Vorrichtungen wie folgt zusammengesetzt:

Abb. 1

Auf einer inerten Trägerfolie 12 ist eine aus Glasfaser bestehende Schicht 4 befestigt, die einerseits zum Transport des Serums und andererseits zur Trennung von Serum und Erythrocyten dient. Diese Schicht 4 teilweise überdeckend ist eine weitere aus Glasfaser bestehende Abtrennzone 5 mittels eines Fixiernetzes 6 befestigt. Auf dieses Netz 6 wird Vollblut aufgetragen, das in der Zone 5 bzw. Zone 4 in Serum und Erythrocyten getrennt wird, wobei letztere festgehalten werden, so dass in den linken Bereich der Zone 4 nur Serum übertritt. Seitlich von der Zone 4 sind über eine Klebeverbindung 13 ein dünnes Kunststoffgewebe 3 sowie eine aus einem durchsichtigen Kunststoff bestehende Trägerfolie 1 befestigt. Unter der Trägerfolie 1 ist wiederum eine Reagenzzone 2 befestigt, die entweder aus einem quellfähigen oder saugfähigen Film besteht und in dem die für die Reaktion notwendigen Reagenzien eingearbeitet sind. Ein Teil der Reagenzien, insbesondere die für eine Vorreaktion notwendigen, können bereits in der Zone 4 enthalten sein. Durch Druck auf die Trägerfolie 1 wird die Reaktion gestartet, nachdem das Serum die Zone 4 vollständig gefüllt hat, welches durch den Druckkontakt durch das Netz 3 in die Reagenzzone 2 eindringt und diese gleichmässig durchfeuchtet. Sollte zusätzlicher Luftsauerstoff für die Reaktion notwendig sein, kann nach Durchfeuchtung der der Zone 2 die Vorrichtung wieder auseinandergetrennt werden. Die Reaktion wird anhand der Verfärbung in der Zone 2 durch die Trägerfolie 1 hindurch beobachtet.

Abb. 2

Der Aufbau der zur Gewinnung des Serums dienenden Zonen entspricht Abb. 1. Um eine Separierung der Reagenzien, die ggf. nicht miteinander lagerstabil sind, zu gewährleisten, sind zwei Reagenzpapiere 8 und 9 vorgesehen, die zusammen mit der schützenden Deckfolie 7 über die Klebestelle 13 mit der Trägerfolie 12 verbunden sind. Wiederum kann nach Sättigung der Zone 4 mit Serum durch Druck auf die Deckfolie 7 ein Flüssigkeitskontakt der Reagenzpapiere mit dem Serum bewirkt werden, der eine Vermischung des Serums und der Reagenzien in den Reagenzpapieren 8 und 9 bewirkt. Die Reaktion findet statt. Sie kann durch die Deckfolie 7 verfolgt werden.

Abb. 3

Diese Vorrichtung entspricht im Grundaufbau wieder der Abb. 1, jedoch ist statt des Zwischengewebes 3 eine optische Sperrschicht 10 vorgesehen. Diese Sperrschicht 10 ist mit Bariumsulfat, Titandioxyd oder ähnlichen stark reflektierenden Substanzen durchsetzt und besteht üblicherweise aus einem Kunststoff- oder Gelatine-Film. Durch diese Schicht 10 wird bewirkt, dass einerseits das zur Beobachtung der Reaktion eingestrahlte Licht vollständig remitiert wird und andererseits eventuelle Verfärbungen der Zone 4 nicht sichtbar werden können.

Abb. 4

Entspricht bezüglich des Serumgewinnungsanteils wiederum Abb. 1. Die Reagenzzone 2, die in diesem Fall aus einem Reagenzfilm besteht, ist in diesem Fall auf die eine Seite eines multifilen Gewebes 11 aufgetragen, welches einerseits zur Stabilisierung des Reagenzfilms dient und andererseits die Benetzung durch das Serum und den Zutritt von Luftsauerstoff fördert. Das Gewebe 11 und die lose aufgelegte Deckfolie 7 sind wiederum mit einer Klebestelle 13 an der Trägerfolie 12 befestigt. Durch Druck auf die Deckfolie 7 wird der Kontakt zwischen dem in der Zone 4 befindlichen Serum und der Reagenzzone 2 hergestellt und die Reaktion gestartet.

Es besteht natürlich auch die Möglichkeit, die erfindungsgemässen Substanzen in Gelatinematrices, gemäss DE-OS 2 735 690, zusammen mit den für die entsprechende Nachweisreaktion benötigten Reagenzien und Hilfsstoffen einzuarbeiten.

Zusammenfassend ist festzustellen, dass die erfindungsgemässen Verbindungen der allgemeinen Formel I in allen Testsystemen einsetzbar sind, mit deren Hilfe direkt oder nach vorangeschalteten Reaktionen Wasserstoffperoxid oder peroxidatisch wirksame Substanzen nachgewiesen werden können.

Die erfindungsgemässen Substanzen der Formel I werden in der Weise hergestellt, dass man in an sich bekannter Weise entweder

a) eine Verbindung der Formel II

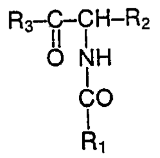 (II)

in der

$R_1$, $R_2$ und $R_3$ die angegebehe Bedeutung haben, mit einer Lewis-Säure oder Pentasulfid umsetzt,

oder

b) für den Fall, dass X Sauerstoff bedeutet, eine Verbindung der Formel III

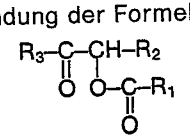 (III)

in der

$R_1$, $R_2$ und $R_3$ die angegebene Bedeutung haben,

mit Ammoniumacetat in Eisessig umsetzt,

oder

c) für den Fall, dass X Schwefel bedeutet, ein Thioamid der Formel IV

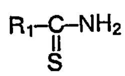 (IV)

in der

$R_1$ die oben angegebene Bedeutung hat, mit einem α-Halogenketon der Formel V

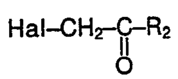 (V)

in der

$R_2$ die angegebene Bedeutung hat und Hal Fluor, Chlor oder Brom darstellt,

umsetzt

und anschliessend, falls gewünscht, die erhaltenen Verbindungen in Verbindungen der Formel I überführt sowie entsprechende Basen in Salze oder Salze in die freien Basen umwandelt.

Im Einzelnen erfolgt die Herstellung der Verbindungen der Formel I wie folgt:

Die Umsetzung von aromatischen α-Acyloxyketonen (Hergestellt durch Acylierung von Acyloinen bzw. Benzoinen mit Säurehalogeniden in Pyridin oder durch Umsetzung von α-Bromketonen mit Salzen von Carbonsäuren in DMF) mit Ammoniumacetat in Eisessig nach der Methode von Davidson führt unter Einführung von Iminostickstoff und Ringschluss zu 2,4-diarylsubstituierten 1,3-Oxazolen. Entsprechende Benzoinester können zur Synthese von 2,4,5-triarylsubstituierten 1,3-Oxazolen herangezogen werden.

Die Cyclisierung von α-Oximinoketonen mit aromatischen Aldehyden unter Einwirkung von Chlorwasserstoffgas in Eisessig liefert bei der nachfolgenden Reduktion der entstandenen 1,3-Oxazol-N-oxide mit Zink in Essigsäure, je nach der Konstitution des Ausgangsmaterials, 2,4- bzw. 2,5-diarylsubstituierte 1,3-Oxazole. Durch Kondensation entsprechender α-Acylaminoketone mit Polyphosphorsäureethylester erhält man analog 2,5-diaryl- bzw. 2,4,5-triarylsubstituierte 1,3-Oxazole [Methode nach Robinson-Gabriel]. 2,5-diarylsubstituierte 1,3-Oxazole sind auch aus Aldehydcyanhydrinen und Aldehyden durch Kondensation mit etherischer Chlorwasserstoffsäure zugänglich (Oxazolsynthese nach Fischer).

Die Umsetzung von 2-subst. 4-Aminophenyl-5-methyl-1,3-oxazol mit Formaldehyd in Methanol führt u.a. zur Bildung des N,N-Bismethylethers, die reduktive Methylierung des Hydrochlorids der gleichen Verbindung mit Formaldehyd in Gegenwart von Platinoxid und katalytisch erregtem Wasserstoff zur N,N-Dimethylverbindung.

Durch Alkylierung von 2-subst. 4-Aminophenyl-5-methyl-1,3-oxazol mit 2,3-Epoxy-1-propanol bzw. Epichlorhydrin erhält man das entsprechende Aminopropandiol resp. das Aminochlorpropanolderivat.

Durch Substitution des Halogens im vorgenannten Aminochlorpropanol durch Umsetzung mit Morpholin entsteht das entsprechende Morpholinderivat. Die Umsetzung des 2-subst. 4-Aminophenyl-5-methyl-1,3-oxazol mit 2,3-Epoxypropanphosphonsäurediethylesters führt zum 1,3-Oxazolphosphonsäurediethylester – dieser lässt sich durch Hydrolyse mit 6 N Chlorwasserstoff leicht in die betreffende Phosphonsäure überführen.

Die reduktive Alkylierung des vorgenannten Phosphonsäureesters mit Formalin führt zum N-methylierten Derivat, dessen Verseifung mit 6 N HCl zur freien Phosphonsäure.

Durch Einwirkung von Bromessigsäurebenzylester auf 2-subst. 4-Aminophenyl-5-methyl-1,3-oxazol erhält man den entsprechenden Eissigsäurebenzylester, und daraus durch katalytische Debenzylierung die betreffende Aminoessigsäure.

Die Umsetzung von 4-Dimethylamino-α-bromacetophenon mit 4-Benzyloxy-3,5-dimethoxyphenylthioamid nach der Methode von Hantzsch und nachfolgende hydrolytische, saure Debenzylierung führt zum diarylsubstituierten 1,3-Thiazol.

Aus Diaryl-α-acylaminoketon erhält man mit Phosphorpentasulfid das entsprechende 2,4,5-triarylsubstituierte 1,3-Thiazol.

Der besseren Schreibweise wegen sind in den Beispielen die Extinktionen als $\varepsilon' = \varepsilon \times 10^{-3}$ angegeben worden.

Bevorzugt im Sinne der Erfindung sind die folgenden Verbindungen:

1. 2-(3,5-Dimethoxy-4-hydroxyphenyl)-4-(4-dimethylaminophenyl)-5-butyl-1,3-oxazol
2. 2-(3,5-Dimethoxy-4-hydroxyphenyl)-4-(4-dimethylaminophenyl)-5-(3-propoxymethyl)-1,3-oxazol

3. 2-(3,5-Dimethoxy-4-hydroxyphenyl)-4-(4-dimethylaminophenyl)-5-(4-n-butoxymethyl)-1,3-oxazol
4. 2-(3,5-Dimethoxy-4-hydroxyphenyl)-4-(4-dimethylaminophenyl)-5-(tert.butyl)-1,3-oxazol
5. 2-(3,5-Dimethoxy-4-hydroxyphenyl)-4-(4-dimethylaminophenyl)-5-(cyclohexyl)-1,3-oxazol
6. 2-(3,5-Dimethoxy-4-hydroxyphenyl)-4-(4-dimethylaminophenyl)-5-benzyl-1,3-oxazol
7. 2-(3,5-Dimethoxy-4-hydroxyphenyl)-4-(1,2,3,4-tetrahydrochinolino-6-N-methyl)-5-methyl-1,3-oxazol
8. 2-(3,5-Dimethoxy-4-hydroxyphenyl)-4-(9-julolidino)-5-methyl-1,3-oxazol
9. 2,4-Bis-(3,5-Dimethoxy-4-hydroxyphenyl)-5-methyl-1,3-oxazol
10. 2,5-Bis-(3,5-dimethoxy-4-hydroxyphenyl)-4-methyl-1,3-oxazol
11. 2-(3,5-Di-tert.butyl-4-hydroxyphenyl)-4-(4-dimethylaminophenyl)-1,3-oxazol
12. 2-(3,5-Di-tert.butyl-4-hydroxyphenyl)-4-(4-dimethylaminophenyl)-5-butyl-1,3-oxazol
13. 2-(3,5-Di-tert.butyl-4-hydroxyphenyl)-4-(4-dimethylaminophenyl)-5-(3-methoxypropyl)-1,3-oxazol
14. 2-(3,5-Di-tert.butyl-4-hydroxyphenyl)-4-(4-dimethylaminophenyl-5-(5-methoxypentyl)-1,3-oxazol
15. 2-(3,5-Di-tert.butyl-4-hydroxyphenyl)-4-(4-dimethylaminophenyl)-5-(tert.butyl)-1,3-oxazol
16. 2-(3,5-Di-tert.butyl-4-hydroxyphenyl)-4-(4-dimethylaminophenyl)-5-cyclohexyl-1,3-oxazol
17. 2-(3,5-Di-tert.butyl-4-hydroxyphenyl)-4-(4-dimethylaminophenyl)-5-benzyl-1,3-oxazol
18. 2-(3,5-Di-tert.butyl-4-hydroxyphenyl)-4-(1,2,3,4-tetrahydrochinolino-6-N-methyl)-5-methyl-1,3-oxazol
19. 2-(3,5-Di-tert.butyl-4-hydroxyphenyl)-4-(9-julolidino)-5-methyl-1,3-oxazol
20. 2-(3,5-Di-tert.butyl-4-hydroxyphenyl)-4-methyl-5-(9-julolidino)-1,3-oxazol
21. 2-(1,2,3,4-Tetrahydrochinolino-6-N-methyl)-4-(3,5-dimethoxy-4-hydroxyphenyl)-5-methyl-1,3-oxazol
22. 2-(9-Julolidino)-4-(3,5-dimethoxy-4-hydroxyphenyl)-5-methyl-1,3-oxazol
23. 2,4-Bis-(4-dimethylaminophenyl)-5-(3,5-dimethoxy-4-hydroxyphenyl)-1,3-oxazol
24. 2-(9-Julolidino)-4,5-bis-(3,5-dimethoxy-4-hydroxyphenyl)-1,3-oxazol
25. 2-(3,5-Di-tert.butyl-4-hydroxyphenyl)-4-(4-dimethylaminophenyl)-5-phenyl-1,3-oxazol
26. 2-(3,5-Di-tert.butyl-4-hydroxyphenyl)-4,5-bis-(9-julolidino)-1,3-oxazol
27. 2,4,5-Tris-(3,5-di-tert.butyl-4-hydroxyphenyl)-1,3-oxazol
28. 2-(4-Dimethylaminophenyl)-4,5-bis-(3,5-di-tert.butyl-4-hydroxyphenyl)-1,3-oxazol
29. 2-(1,2,3,4-Tetrahydrochinolino-6-N-methyl)-4,5-bis-(3,5-di-tert.butyl-4-hydroxyphenyl)-1,3-oxazol
30. 2-(9-Julolidino)-4,5-bis-(3,5-di-tert.butyl-4-hydroxyphenyl)-1,3-oxazol
31. 2-(4-Dimethylaminophenyl)-4-(3,5-di-tert.-butyl-4-hydroxyphenyl)-5-methyl-1,3-oxazol
32. 2-(1,2,3,4-Tetrahydrochinolino-6-N-methyl)-4-(3,5-di-tert.butyl-4-hydroxyphenyl)-5-methyl-1,3-oxazol
33. 2-(9-Julolidino)-4-(3,5-di-tert.butyl-4-hydroxyphenyl)-5-methyl-1,3-oxazol
34. 2-(3,5-Dimethoxy-4-hydroxyphenyl)-4,5-bis-(1,2,3,4-tetrahydrochinolino-6-N-methyl)-1,3-oxazol
35. 2-(3,5-Dimethoxy-4-hydroxyphenyl)-4,5-bis-(9-julolidino)-1,3-oxazol
36. 2-(3,5-Dimethoxy-4-hydroxyphenyl)-4-(4-dimethylaminophenyl)-5-phenyl-1,3-oxazol
37. 2-(3,5-Dimethoxy-4-hydroxyphenyl)-4,5-bis-(3,5-di-tert.butyl-4-hydroxyphenyl)-1,3-oxazol
38. 2-(1,2,3,4-Tetrahydrochinolino-6-N-methyl)-4,5-bis-(3,5-dimethoxy-4-hydroxyphenyl)-1,3-oxazol
39. 2-(3,5-Dimethoxy-4-hydroxyphenyl)-4-[4-(N-2,3-dihydroxypropyl)-aminophenyl]-5-methyl-1,3-oxazol
40. 2-(3,5-Dimethoxy-4-hydroxyphenyl)-4-[4-(N-2,3-dihydroxypropyl)-methylaminophenyl]-5-methyl-1,3-oxazol
41. N-〈4-[2-(3,5-Dimethoxy-4-hydroxyphenyl)-5-methyl-4-(1,3)-oxazolyl]-phenylamino〉 methanphosphonsäure
42. N-〈4-[2-(3,5-Dimethoxy-4-hydroxyphenyl)-5-methyl-4-(1,3)-oxazolyl]-methylphenylamino〉 methanphosphonsäure
43. N-〈4-[2-(3,5-Dimethoxy-4-hydroxyphenyl)-5-methyl-4-(1,3)-oxazolyl]-phenylamino〉 ethansulfonsäure
44. N-〈4-[2-(3,5-Dimethoxy-4-hydroxyphenyl)-5-methyl-4-(1,3)-oxazolyl]-phenylamino〉ethanposphonsäure
45. N-〈4-[2-(3,5-Dimethoxy-4-hydroxyphenyl)-5-methyl-4-(1,3)-oxazolyl]-phenylamino〉 methanphosphonsäure
46. N-〈4-[2-(3,5-Dimethoxy-4-hydroxyphenyl)-5-methyl-4-(1,3)-oxazolyl]-methylphenylamino〉 methansulfonsäure
47. N-〈4-[2-(3,5-Dimethoxy-4-hydroxyphenyl)-5-methyl-4-(1,3)-oxazolyl]-methylphenylamino〉 ethanphosphonsäure
48. N-〈4-[2-(3,5-Dimethoxy-4-hydroxyphenyl)-5-methyl-4-(1,3)-oxazolyl]-phenylamino〉essigsäure
49. N,N-〈4-[2-(3,5-Dimethoxy-4-hydroxyphenyl)-5-methyl-4-(1,3)-oxazolyl]-phenylamino〉-bis-ethansulfonsäure
50. N-〈4-[2-(3,5-Dimethoxy-4-hydroxyphenyl)-5-methyl-4-(1,3-oxazolyl]-phenylamino〉-bis-methanphosphonsäure
51. N,N-〈4-[2-(3,5-Dimethoxy-4-hydroxyphenyl)-5-methyl-4-(1,3)-oxazolyl]-phenylamino〉-bis-essigsäure
52. 〈4-[2-(3,5-Dimethoxy-4-hydroxyphenyl)-5-methyl-4-(1,3)-oxazolyl]-1,2,3,4-tetrahydrochinolino〉-6-N-ethansulfonsäure
53. 〈2-[4-(3,5-Dimethoxy-4-hydroxyphenyl)-5-methyl-4-(1,3)-oxazolyl]-1,2,3,4-tetrahydrochinolino〉-6-N-ethansulfonsäure
54. 〈4-[2-(3,5-Dimethoxy-4-hydroxyphenyl)-5-methyl-4-(1,3)-oxazolyl]-1,2,3,4-tetrahydrochinolino〉-6-N-ethanphosphonsäure
55. 〈2-[4-(3,5-Dimethoxy-4-hydroxyphenyl)-5-methyl-4-(1,3)-oxazolyl]-1,2,3,4-tetrahydrochinolino〉-6-N-ethanphosphonsäure
56. 〈4-[2-(3,5-Dimethoxy-4-hydroxyphenyl)-5-methyl-4-(1,3)-oxazolyl]-1,2,3,4-tetrahydrochinolino〉-6-N-essigsäure
57. 〈2-[4-(3,5-Dimethoxy-4-hydroxyphenyl)-5-methyl-4-(1,3)-oxazolyl]-1,2,3,4-tetrahydrochinolino〉-6-N-essigsäure
58. 2-(3,5-Dimethoxy-4-hydroxyphenyl)-4-(4-dimethylaminophenyl)-5-hydroxymethyl-1,3-oxazol
59. 〈5-[2-(3,5-Dimethoxy-4-hydroxyphenyl)-4-(4-dimethylaminophenyl)-(1,3)-oxazolyl]-methyl〉trimethylammoniumchlorid
60. 1-〈5-[2-(3,5-Dimethoxy-4-hydroxyphenyl)-4-(4-dimethylaminophenyl)-(1,3)-oxazolyl]-methyl〉pyridiniumchlorid
61. 2-(3,5-Di-tert.butyl-4-hydroxyphenyl)-4-[4-N-3-chlor-2-hydroxypropyl-aminophenyl]-5-methyl-1,3-oxazol
62. 2-(3,5-Di-tert.butyl-4-hydroxyphenyl)-4-[4-(N-2,3-dihydroxypropyl)-aminophenyl]-5-methyl-1,3-oxazol
63. 2-(3,5-Di-tert.butyl-4-hydroxyphenyl)-4-[4-(N-2,3-dihydroxypropyl)-methylaminophenyl]-5-methyl-1,3-oxazol
64. N-〈4-[2-(3,5-Di-tert.butyl-4-hydroxyphenyl)-5-methyl-4-(1,3)-oxazolyl]-phenylamino〉methanphosponsäure
65. N-〈4-[2-(3,5-Di-tert.butyl-4-hydroxyphenyl)-5-methyl-4-(1,3)-oxazolyl]-methylphenylamino)-methanphosphonsäure
66. 2-(3,5-Di-tert.butyl-4-hydroxyphenyl)-4-[4-(3-N-morpholino-2-hydroxypropyl)-aminophenyl]-5-methyl-1,3-oxazol
67. N-〈4-[2-(3,5-Di-tert.butyl-4-hydroxyphenyl)-5-methyl-4-(1,3)-oxazolyl]-phenylamino-2-hydroxypropyl〉phosphonsäurediethylester
68. N-〈4-[2-(3,5-Di-tert.butyl-4-hydroxyphenyl)-5-methyl-4-(1,3)-oxazolyl]-phenylamino-2-hydroxypropyl〉phosphonsäure
69. N-〈4-[2-(3,5-Di-tert.butyl-4-hydroxyphenyl)-5-methyl-4-(1,3)-oxazolyl]-methylphenylamino-2-hydroxypropyl〉phosphonsäure
70. N-〈4-[2-(3,5-Di-tert.butyl-4-hydroxyphenyl)-5-methyl-4-(1,3)-oxazolyl]phenylamino-ethansulfonsäure
71. N-〈4-[2-(3,5-Di-tert.butyl-4-hydroxyphenyl)-5-methyl-4-(1,3)-oxazolyl]phenylamino〉ethanphosphonsäure
72. N-〈4-[2-(3,5-Di-tert.butyl-4-hydroxyphenyl)-5-methyl-4-(1,3)-oxazolyl]phenylamino〉methanphosphonsäure
73. N-〈4-[2-(3,5-Di-tert.butyl-4-hydroxyphenyl)-5-methyl-4-(1,3)-oxazolyl]methylphenylamino〉methansulfonsäure
74. N-〈4-[2-(3,5-Di-tert.butyl-4-hydroxyphenyl)-5-methyl-4-(1,3)-oxazolyl]methylphenylamino〉ethanphosphonsäure
75. N,N-〈4-[2-(3,5-Di-tert.butyl-4-hydroxyphenyl)-5-methyl-4-(1,3)-oxazolyl]phenylamino〉-bis-ethansulfonsäure
76. N,N-〈4-[2-(3,5-Di-tert.butyl-4-hydroxyphenyl)-5-methyl-4-(1,3)-oxazolyl]phenylamino〉-bis-methanphosphonsäure
77. N,N-〈4-[2-(3,5-Di-tert.butyl-4-hydroxyphenyl)-5-methyl-4-(1,3)-oxazolyl]phenylamino〉-bis-essigsäure
78. 〈4-[2-(3,5-Di-tert.butyl-4-hydroxyphenyl)-5-methyl-4-(1,3)-oxazolyl]-1,2,3,4-tetrahydrochinolino〉-6-N-ethansulfonsäure
79. 〈2-[4-(3,5-Di-tert.butyl-4-hydroxyphenyl)-5-methyl-4-(1,3)-oxazolyl]-1,2,3,4-tetrahydrochinolino〉-6-N-ethansulfonsäure
80. 〈4-[2-(3,5-Di-tert.butyl-4-hydroxyphenyl)-5-methyl-4-(1,3)-oxazolyl]-1,2,3,4-tetrahydrochinolino〉-6-N-ethanphosphonsäure
81. 〈2-[4-(3,5-Di-tert.butyl-4-hydroxyphenyl)-5-methyl-4-(1,3)-oxazolyl]-1,2,3,4-tetrahydrochinolino〉-6-N-ethanphosphonsäure
82. 〈4-[2-(3,5-Di-tert.butyl-4-hydroxyphenyl)-5-methyl-4-(1,3)-oxazolyl]-1,2,3,4-tetrahydrochinolino〉-6-N-essigsäure
83. 〈2-[4-(3,5-Di-tert.butyl-4-hydroxyphenyl)-5-methyl-(1,3)-oxazolyl]-1,2,3,4-tetrahydrochinolino〉-6-N-essigsäure
84. 2-(3,5-Di-tert.butyl-4-hydroxyphenyl)-4-(4-dimethylaminophenyl)-5-hydroxymethyl-1,3-oxazol
85. 〈5-[2-(3,5-Di-tert.butyl-4-hydroxyphenyl)-4-(4-dimethylaminophenyl)-(1,3)-oxazolyl]-methyl〉trimethylammoniumchlorid
86. 1-〈5-[2-(3,5-Di-tert.butyl-4-hydroxyphenyl)-4-(4-dimethylaminophenyl)-(1,3)-oxazolyl]-methyl〉pyridiniumchlorid
87. 〈5-[4-(4-Dimethylaminophenyl)-5-methyl-2-(1,3)-oxazolyl]-2-hydroxy-3-methoxy〉phenoxy-essigsäure
88. 〈5-[4-(4-Dimethylaminophenyl)-5-methyl-2-(1,3)-oxazolyl]-2-hydroxy-3-tert.butyl〉phenoxy-essigsäure
89. 〈5-[2-(4-Dimethylaminophenyl)-5-methyl-4-(1,3)-oxazolyl]-2-hydroxy-3-methoxy〉-phenoxy-essigsäure
90. 〈5-[2-(4-Dimethylaminophenyl)-5-methyl-4-(1,3)-oxazolyl]-2-hydroxy-3-tert.butyl〉-phenoxy-essigsäure
91. 2-(3,5-Di-tert.butyl-4-hydroxyphenyl)-4,5-bis-(1,2,3,4-tetrahydrochinolino-6-N-methyl)-1,3-oxazol

Beispiel 1

2-(3,5-Dimethoxy-4-hydroxyphenyl)-4-(4-dimethylaminophenyl)-1,3-oxazol-hydrochlorid

a) 4-N,N-Dimethylamino-α-[(3,5-dimethoxy-4-hydroxy)-benzoyloxy]-acetophenon

Die Suspension von 48 g (0,2 Mol) 4-Dimethylamino-α-bromacetophenon und 48,4 g (0,22 Mol) Natriumsyringat in 1,2 l absolutem Dimethylformamid werden unter Rühren zwei Stunden auf 130 °C erwärmt. Nach Einengen des Lösungsmittels im Vakuum auf ca. ein Drittel seines Volumens gibt man 1 l Eiswasser zu, saugt die gebildeten Kristalle ab, wäscht den Filterkuchen mit 500 ml

Wasser und kristallisiert das Rohprodukt aus 400 ml Essigsäure um. Nach Absaugen wäscht man das Kristallisat mit je 200 ml Wasser und Aceton. Anschliessend trocknet man bei 40 °C im Vakuum und erhält 42,1 g (55,6% Ausbeute) der Titelverbindung, beigefarbene Kristalle, Fp. 208–210 °C.

b) 36 g (0,1 Mol) des unter a) isolierten Esters werden in 500 ml Eisessig mit 38,6 g (0,5 Mol) Ammoniumacetat unter Rühren zwei Stunden auf 130 °C erhitzt. Nach dem Erkalten giesst man auf 2 l Eiswasser, saugt das Kristallisat ab, wäscht mit Wasser und Ether, dabei erhält man 15,1 g beigefarbene Kristalle. Das Rohprodukt wird säulenchromatographisch an Kieselgel 60 mit Chloroform-Methanol 9:1 gereinigt. Die entsprechende Fraktion gibt beim Einengen 7,3 g (20,6% Ausbeute) der Titelverbindung, Fp. 202–204 °C (Zers.).

$\lambda max = 498$ nm, $\varepsilon' = 20{,}5$ $cm^2$ $\mu mol^{-1}$

Analog werden erhalten:

1.1. 2,4-Bis-(3,5-dimethoxy-4-hydroxyphenyl)-1,3-oxazol

a) 3,5-Dimethoxy-4-hydroxy-α-[(3,5-dimethoxy-4-hydroxy)-benzoyloxy]acetophenon, Ausbeute 94%, Fp. 147/153 °C

b) Titelverbindung (Base), Ausbeute 29%, Fp. 168–172 °C

Hydrochlorid, Fp. 132–134 °C (Zers.)

$\lambda max.$ 470 nm, $\varepsilon' = 14{,}4$ $cm^2$ $\mu mol^{-1}$

1.2. 2-(4-Dimethylaminophenyl)-4-(3,5-dimethoxy-4-hydroxyphenyl)-1,3-oxazol-hydrochlorid

a) 4-Benzyloxy-3,5-dimethoxy-α-(4-dimethylamino-benzoyloxy)-acetophenon. Ausbeute 53%, Fp. 114 °C

b) 2-(4-Dimethylaminophenyl)-4-(4-benzyloxy-3,5-dimethoxyphenyl)-1,3-oxazol. Ausbeute 16%, Fp. 157 °C

c) 3,4 g (0,01 Mol) des unter b) erhaltenen Oxazolderivates werden in 100 ml Ethanol nach Zusatz von 0,3 g 10% Palladium auf Aktivkohle bei 25 °C unter Normaldruck hydriert. Abfiltrieren des Katalysators und Einengen ergibt:

2,7 g der Titelverbindung (Ausbeute 65% d.Th.), Fp. 185 °C

$\lambda max.$ 512 nm, $\varepsilon' = 5{,}3$ $cm^2$ $\mu mol^{-1}$

1.3. 2-(4-Dimethylaminophenyl)-4-(3,5-dimethoxy-4-hydroxyphenyl)-5-methyl-1,3-oxazol-hydrochlorid

a) 3,5-Dimethoxy-4-hydroxy-α-(4-dimethylaminobenzoyloxy)-propiophenon (Ausbeute 40,5%). Fp. 152–155 °C (Zers.)

b) Titelverbindung, Ausbeute 20,5% d.Th., Fp. 128 °C

$\lambda max.$ 327 nm, $\varepsilon' = 78{,}9$ $cm^2$ $\mu mol^{-1}$

1.4. 2-(9-Julolidino)-4-(3,5-dimethoxy-4-hydroxyphenyl)-1,3-oxazol-hydrochlorid

a) 3,3-Dimethoxy-4-hydroxy-α-(9-julolidinocarboxy)-acetophenon, Reinigung durch Chromatographie an Kieselgel. Laufmittel: Chloroform, Ausbeute 42,8% d.Th., amorphes Pulver.

DC: Kieselgelplatte, Laufmittel: n-Heptan-Methylethylketon 1:1

RF-Wert: 0,48

b) Titelverbindung (Ausbeute 25,6% d.Th.), farblose Kristalle, Fp. 231–233 °C

$\lambda max.$ 540 nm, $\varepsilon' = 21{,}6$ $cm^2$ $\mu mol^{-1}$

1.5. 2-(3,5-Di-tert.butyl-4-hydroxyphenyl)-4-(4-dimethylamino-phenyl)-5-methyl-1,3-oxazol

a) 4-Dimethylamino-α-([3,5-di-tert.butyl-4-hydroxy]-benzoyloxy)propiophenon, Ausbeute 86,5% d.Th., Fp. 172 °C

b) Titelverbindung: Ausbeute 95%, Fp. 125 °C (Zers.)

$\lambda max$ 488 nm, $\varepsilon' = 46{,}4$ $cm^2$ $\mu mol^{-1}$

1.6. 2-(3,5-Di-tert.butyl-4-hydroxyphenyl)-4-(3,5-dimethoxy-4-hydroxyphenyl)-5-methyl-1,3-oxazol

a) 4-(3,5-Dimethoxy-4-hydroxyphenyl)-α-(3,5-di-tert.butyl-4-hydroxy)benzoyloxy-propiophenon, Ausbeute 90,5%, amorphes gelbliches Pulver.

DC-Kieselgelplatte, Laufmittel: Toluol-Essigester 5:1

RF-Wert 0,425

b) Titelverbindung: Ausbeute 82,4%, Fp. 83–85 °C

$\lambda max = 486$ nm, $\varepsilon' = 31{,}1$ $cm^2$ $\mu mol^{-1}$

1.7. 2-(3,5-Dimethoxy-4-hydroxyphenyl)-4-(4-dimethylaminophenyl)-5-phenyl-1,3-oxazol

a) 4-Dimethylamino-O-(4-benzyloxy-3,5-dimethoxybenzoyl)-benzoin, Fp. 126–128 °C

b) 2-(3,5-Dimethoxy-4-benzyloxyphenyl)-4-(4-dimethylaminophenyl)-5-phenyl-1,3-oxazol (direkt weiterverarbeitet)

c) Anstelle der in Beispiel 1.2 c erwähnten katalytischen Debenzylierung wird die O-Benzylschutzgruppe hier nach Ringschluss von a) zum 1,3-Oxazol b) durch 30′ Kochen mit 6 N Salzsäure abgespalten.

Titelverbindung: Ausbeute 40%, Fp. 167 °C (Zers.)

$\lambda max$ 582 nm, $\varepsilon' = 36{,}4$ $cm^2$ $\mu mol^{-1}$

1.8. 2-(3,5-Di-tert.butyl-4-hydroxyphenyl)-4-(9-julolidino)-5-methyl-1,3-oxazol

a) 9-Julolidino-α-brommethylketon

Aus 9-Julolidinaldehyd erhält man durch Umsetzung mit Ethylmagnesiumbromid nach Grignard das entsprechende Carbinol, Fp. 74–77 °C. Die Oxidation dieses Produktes nach Oppenauer ergibt 9-Julolidinethylketon, Fp. 68,5 °C–69,5 °C aus dem durch Bromierung in Eisessig 9-Julolidin-α-brom-ethyl-keton, Fp. 82–84 °C erhalten wird.

b) 9-Julolidin-α-(3,5-di-tert.butyl-4-hydroxy)-benzoyloxyethylketon, Fp. 165–170 °C

c) Titelverbindung, Ausbeute 34%, Fp. 135 °C (Zers.)

$\lambda max$ 490 nm, $\varepsilon' = 35{,}2$ $cm^2$ $\mu mol^{-1}$

Beispiel 2

2,4,5-Tris-(3,5-dimethoxy-4-hydroxyphenyl)-1,3-oxazol-hydrochlorid

a) 3,3′,5,5′-Tetramethoxy-4,4′-dibenzyloxybenzoin. 81,7 g (0,3 Mol) 4-Benzyloxy-3,5-dimethoxybenzaldehyd werden in 300 ml Ethanol suspendiert, 6 g Kaliumcyanid zugegeben und drei Stunden unter Rückfluss erhitzt, wobei der Aldehyd in Lösung geht. Danach giesst man auf 1,5 l Eiswasser und rührt zwei Stunden unter Eiskühlung nach, nimmt das erhaltene Rohprodukt mit 2 × 300 ml Dichlormethan auf, trocknet über Natriumsulfat und engt im Vakuum ein. Der Rückstand, 87,3 g öliges Material, wird durch Chromatographie an Kieselgel gereinigt. Die entsprechende Fraktion wird eingeengt, 52,4 g (Ausbeute 63,2% d.Th.), gelbliche Kristalle der Titelverbindung. Fp. 149–150 °C.

b) 3,3',5,5'-Tetramethoxy-4,4'-dibenzyloxy-O-(4-benzyloxy-3,5-dimethoxy-benzoyl)-benzoin

15,8 g (0,029 Mol) des vorgenannten Benzoins werden in 40 ml Pyridin suspendiert und unter Rühren eine Lösung von 13,4 g (0,0435 Mol) 4-Benzyloxy-3,5-dimethoxybenzoylchlorid in 80 ml absolutem Pyridin zugetropft. Nach acht Stunden Stehen bei Raumtemperatur engt man am Rotationsverdampfer im Vakuum ein, nimmt den Rückstand in 100 ml Dichlormethan auf, schüttelt die Lösung nacheinander mit 2 N HCl, $H_2O$, 2 N $Na_2CO_3$, und Wasser durch und engt im Vakuum ein. Man erhält 25 g ölige Titelverbindung,

DC: Kieselgel 60, Laufmittel:

Isopropanol-n-Butylacetat-$H_2O$-Ammoniak 5:2:1,5:0,5

c) 2,4,5-Tris-(4-Benzyloxy-3,5-dimethoxyphenyl)-1,3-oxazol

Man erhitzt 20 g (0,0245 Mol) der unter b) vorgenannten Verbindung mit 28,4 g (0,37 Mol) Ammoniumacetat in 250 ml Eisessig fünf Stunden unter Rückfluss, danach giesst man auf ein Gemisch von 500 ml Wasser und 250 ml konz. Ammoniak; das abgeschiedene kristalline Rohprodukt wird abfiltriert, mit Wasser gewaschen und getrocknet. Ausbeute 24,6 g. Zur Reinigung chromatografiert man an Kieselgel. Laufmittel Toluol-Essigester 4:1. Man erhält 13 g (Ausbeute 66,4% d.Th.), beigefarbene Kristalle der Titelverbindung, Fp > 80 bis 120 °C (kein klarer Schmelzpunkt).

DC: Kieselgel, Laufmittel: Toluol-Essigester 4:1

RF-Wert: 0,64

d) 11 g (0,014 Mol) des vorstehenden Oxazols werden in 300 ml Methanol gelöst und unter Zugabe von 1,1 g Palladium auf Kohle (10%ig) bei Normaldruck hydriert. Nach Aufnahme der berechneten Menge Wasserstoff filtriert man den Katalysator ab und engt das Filtrat nach Zugabe von 7,5 ml 5 N etherischer Chlorwasserstoffsäure im Vakuum ein. Man erhält 7 g (Ausbeute 82,1% d.Th.) der Titelverbindung, farblose Kristalle, Fp. 205–207 °C (Zers.).

$\lambda$max 632 nm, $\varepsilon' = 22{,}2\ cm^2\ \mu mol^{-1}$

2.1. 2-(3,5-Di-tert.butyl-4-hydroxyphenyl)-4,5-bis-(3,5-dimethoxy-4-hydroxyphenyl)-1,3-oxazol

a) 3,3',5,5'-Tetramethoxy-4,4'-dibenzyloxy-α-(4-hydroxy-3,5-di-tert.butyl)benzoyloxy-benzoin, Ausbeute 42,5%, gelbliches amorphes Pulver

DC: Kieselgel 60, Laufmittel: Toluol-Essigester 5:1

RF-Wert: 0,61

b) 2-(3,5-Di-tert.butyl-4-hydroxyphenyl)-4,5-bis(3,5-dimethoxy-4-benzyloxyphenyl)-1,3-oxazol, Ausbeute 62,5%, rötliches Öl

DC: Kieselgel 60, Laufmittel: Toluol-Essigester 5:1

RF-Wert: 0,56

c) Titelverbindung: Ausbeute 81,5%, Fp. 120 °C (Zers.)

$\lambda$max 587 nm, $\varepsilon' = 40{,}2\ cm^2\ \mu mol^{-1}$

Beispiel 3

2-(3,5-Dimethoxy-4-hydroxyphenyl)-4-(4-aminophenyl)-5-methyl-1,3-oxazol-hydrochlorid

a) 2-(3,5-Dimethoxy-4-hydroxyphenyl)-4-acetaminophenyl-5-methyl-1,3-oxazol-N-oxid

Man suspendiert 50 g (0,227 Mol) 4-Acetaminophenyl-1-oximino-2-propanon und 41,35 g (0,227 Mol) 3,5-Dimethoxy-4-hydroxybenzaldehyd in 600 ml Eisessig und leitet unter Rühren fünf Stunden trockenes Chlorwasserstoffgas durch die Reaktionsmischung, danach engt man im Vakuum auf die Hälfte ein. Nach Zugabe von 200 ml Ethanol filtriert man ab und wäscht den Filterkuchen mit 150 ml Ethanol. Ausbeute 75,6 g = 80,2%, bräunliche Kristalle, der Titelverbindung, Fp. > 200 °C (Zers.)

DC: Kieselgel, Laufmittel: Chloroform-Methanol 8:1

RF-Wert: 0,31

b) 75,62 g (0,182 Mol) des vorgenannten Produkts werden in 900 ml Eisessig suspendiert und unter Rühren 220 g (3,35 g-Atom) Zinkstaub portionsweise zugegeben. Danach wird zwei Stunden unter Rückfluss erhitzt, heiss abfiltriert, das Filtrat im Vakuum eingeengt und mit 500 ml 6 N HCl 30 min unter Rückfluss gekocht. Man kühlt mit Eis, saugt die gebildeten Kristalle ab und wäscht den Filterrückstand mit 150 ml Aceton. Das zinkhaltige Rohprodukt wird in 1,2 l heissem Wasser gelöst, mit einer Lösung von 55 g Titriplex III in 1,5 l Wasser versetzt und die Base durch Zugabe von 50 ml konz. Ammoniak in Freiheit gesetzt. Nach Abfiltrieren und Waschen des Filterrückstandes mit Wasser kristallisiert man aus 6 N HCl um und erhält nach Abfiltrieren, Waschen des Hydrochlorids mit Aceton und Trocknen im Vakuum bei 40 °C 60,2 g (79,8% Ausbeute) der Titelverbindung, Fp. 253–255 °C (Zers.)

$\lambda$max. 540 nm, $\varepsilon' = 29\ cm^2\ \mu mol^{-1}$

In analoger Weise werden erhalten:

3.1. 2-(3,5-Dimethoxy-4-hydroxyphenyl-4-(4-amino-3-methoxyphenyl)-5-methyl-1,3-oxazol-hydrochlorid

aus 4-Acetamino-3-methoxyphenyl-1-oximino-2-propanon und 3,5-Dimethoxy-4-hydroxybenzaldehyd, Fp. > 227 °C (Zers.), gelbliche Kristalle

$\lambda$max. 546 nm, $\varepsilon' = 21{,}6\ cm^2\ \mu mol^{-1}$

3.2. 2-(3,5-Dimethoxy-4-hydroxyphenyl)-4-methyl-5-(4-dimethylamino-phenyl)-1,3-oxazol-hydrochlorid

aus 4-Dimethylaminophenyl-2-oximino-1-propanon und 3,5-Dimethoxy-4-hydroxybenzaldehyd, Fp. 168–170 °C (Zers.), farblose Kristalle

$\lambda$max. 494 nm, $\varepsilon' = 5{,}7\ cm^2\ \mu mol^{-1}$

3.3. 2-(3,5-Di-tert.butyl-4-hydroxyphenyl)-4-(4-aminophenyl)-5-methyl-1,3-oxazol-hydrochlorid

aus 4-Acetaminophenyl-1-oximino-2-propanon und 3,5-Di-tert.butyl-4-hydroxybenzaldehyd über das Oxim:

[Fp. 215 °C (Zers.)] → farblose Kristalle, Fp. 238 °C

DC: Kieselgel 60, Laufmittel: Chloroform-Methanol 19:1

RF-Wert: 0,65 (Base)

λmax 514 nm, ε' = 41,6 $cm^2$ $\mu mol^{-1}$

3.4. 2-(4-Dimethylaminophenyl)-bis-4,5-(3,5-dimethoxy-4-hydroxyphenyl)-1,3-oxazol-hydrochlorid

a) Bis-(3,3',5,5')-Tetramethoxy-4,4'-benzyloxy-benzilmonoxim

Man suspendiert 27,13 g (0,05 Mol) Bis-(3,3',5,5')-Tetramethoxy-4,4'-benzyloxybenzil in 350 ml trockenem Pyridin, gibt 6,35 g (0,1 Mol) Hydroxylaminhydrochlorid zu und rührt 4,5 Stunden bei Raumtemperatur, danach engt man im Vakuum ein und trennt den Rückstand chromatografisch an einer Kieselgelsäule. Laufmittel: Toluol-Essigester 2:1. Die entsprechende Fraktion enthält 17,3 g (62,2% Ausbeute) Oxim, rötliches Öl.

DC: Kieselgel 60, Laufmittel: Toluol-Essigester 2:1

RF-Wert: 0,64

b) 14,49 g (0,026 Mol) der vorstehenden Verbindung werden gemeinsam mit 3,88 g (0,026 Mol) 4-Dimethylaminobenzaldehyd in 50 ml Eisessig gelöst und bei 15–20 °C 3,5 Stunden unter Rühren trockenes Chlorwasserstoffgas eingeleitet. Danach engt man im Vakuum weitgehend ein, rührt den Rückstand mit Ether an und reinigt das erhaltene Rohprodukt an Kieselgel. Laufmittel: Dichlormethan-Aceton-Methanol 20:10:2. Man erhält 3,1 g (21,1% Ausbeute), beigefarbene Kristalle, Fp. 212–215 °C (Zers.).

λmax. 619 nm, ε' = 25,9 $cm^2$ $\mu mol^{-1}$

Beispiel 4

2-(3,5-Dimethoxy-4-hydroxyphenyl)-4,5-bis-(4-dimethylaminophenyl)-1,3-oxazol

a) 4,4'-Bis-(dimethylamino)-benzilmonoxim

74 g (0,25 Mol) 4,4'-Bis-(dimethylamino)-benzil werden mit 20,8 g (0,3 Mol) Hydroxylaminohydrochlorid in 1,2 l Pyridin 30 Stunden bei Raumtemperatur gerührt, im Vakuum zur Trockne eingeengt, mit 500 ml Wasser, anschliessend mit 500 ml einer Mischung Methanol-Wasser 1:3 und schliesslich mit 500 ml Toluol angerührt, abgesaugt und getrocknet. Man erhält 69,6 g (89,2% Ausbeute) 4,4'-Bis-(dimethylamino)-benzilmonoxim, Fp. 179 °C (Zers.).

b) 4,4'-Bis-(dimethylamino)-2-amino-desoxybenzoin-hydrochlorid

10 g (0,032 Mol) der vorstehenden Verbindung werden in 60 ml 6 N methanolischer HCl bei Raumtemperatur mit 1,93 g Palladium auf Kohle hydriert. Nach beendeter Wasserstoffaufnahme wird der Katalysator abgesaugt und die Lösung im Vakuum eingedampft. Nach Umkristallisieren aus Methanol ergeben sich 9,1 g (84,6% Ausbeute), gelbliche Kristalle der Titelverbindung, Fp. 218–220 °C (Zers.).

c) 4,4'-Bis-(dimethylamino)-2-(4-benzyloxy-3,5-dimethoxybenzamino)-desoxybenzoin

33,74 g (0,11 Mol) 4-Benzyloxy-3,5-dimethoxybenzoylchlorid werden in 250 ml abs. Pyridin gelöst, dann eine Suspension von 33,4 g (0,1 Mol) der unter b) erhaltenen Verbindung in 300 ml abs. Pyridin bei 5–10 °C eingetropft, 8 Stunden bei 40 °C gerührt und im Vakuum eingeengt. Der Rückstand wird in 200 ml Dichlormethan aufgenommen, dreimal mit je 100 ml 2 N HCl, 100 ml Wasser, 100 ml Natriumhydrogenkarbonatlösung und 100 ml Wasser durchgeschüttelt, über Natriumsulfat getrocknet und eingedampft. Das Rohprodukt, 53,2 g halbkristallines Material, wird in einer Mischung von 650 ml Toluol-Methanol 12:1 heiss gelöst und das anfallende ölige Produkt durch Anreiben mit Ether zur Kristallisation gebracht. Man isoliert 33,1 g (58,3% Ausbeute) beigefarbene Kristalle, Fp. 207 °C (Zers.) der Titelverbindung.

d) 2-(3,5-Dimethoxy-4-benzyloxyphenyl)-4,5-bis-[4-(dimethylaminophenyl)]-1,3-oxazol

28,3 g (0,05 Mol) der vorgenannten Verbindung werden mit 243 g (0,75 Mol) Polyphosphorsäureethylester in 300 ml wasserfreiem Chloroform sieben Stunden unter Rückfluss erhitzt, danach unter Zugabe von 6 N Ammoniak neutralisiert, das Reaktionsprodukt mit Dichlormethan extrahiert, die organische Phase mit Wasser durchgeschüttelt, über Natriumsulfat getrocknet und eingeengt. Die Titelverbindung, 30 g braunes Öl, wird direkt, ohne Reinigung, für die nächste Stufe eingesetzt.

DC: Kieselgel 60, Laufmittel: Chloroform-Essigester 4:1

RF-Wert: 0,66

e) 28 g (0,05 Mol) des nach d) erhaltenen Rohprodukts werden mit 200 ml 6 N HCl eine Stunde unter Rückfluss gekocht. Danach engt man im Vakuum ein und kristallisiert den Rückstand zweimal aus Methanol-Aceton 2:1 um. Die schwach gelbliche Titelverbindung wird abfiltriert, der Filterkuchen mit Aceton gewaschen und im Vakuum bei 50 °C getrocknet. Ausbeute 16,1 g (35,1% d.Th.), farblose Kristalle, Fp. 232–234 °C (Zers.).

λmax. 550 nm, ε' = 8,57 $cm^2$ $\mu mol^{-1}$

4.1. 2-(3,5-Dimethoxy-4-hydroxyphenyl)-5-(4-dimethylaminophenyl)-1,3-oxazol-hydrochlorid

a) 4-Dimethylamino-ω-aminoacetophenonhydrochlorid

6 g (0,03 Mol) 4-Dimethylamino-ω-hexamethylentetraminiumacetophenonbromid werden in 28 ml Ethanol suspendiert und nach Zugabe von 14 ml konz. Salzsäure 3 Tage bei Raumtemperatur gerührt, danach abfiltriert und das Filtrat im Vakuum eingeengt. Man erhält 5 g 4-Dimethylamino-ω-aminoacetophenonhydrochlorid, Fp. 185 °C (Zers.).

b) 4-Dimethylamino-ω-(4-benzyloxy-3,5-dimethoxybenzamido)-acetophenon

4,81 g vorstehender Verbindung werden in 50 ml abs. Pyridin suspendiert und eine Lösung von 7,56 g (0,025 Mol) 4-Benzyloxy-3,5-dimethoxybenzoylchlorid in 70 ml abs. Pyridin unter Eiskühlung zugetropft. Nach 6 Stunden wird abfiltriert und im Vakuum eingedampft. Nach Aufnehmen in Dichlormethan wird mit Wasser und 2 N HCl durchgeschüttelt, die organische Phase getrocknet, im Vakuum eingeengt und der Rückstand mit

50 ml Ether angerieben. Es ergeben sich 7,11 g der Titelverbindung, Fp. 128–131 °C (Zers.).

c) 5 g (0,01 Mol) der Verbindung b) werden mit 54,4 g (0,16 Mol) Polyphosphorsäureethylester in 300 ml abs. Chloroform 5 Stunden unter Argon am Rückfluss erhitzt. Anschliessend wird am Rotationsverdampfer eingeengt. Man erhält ein leicht bräunliches Öl, dieses wird mit 150 ml 6 N HCl 20 Minuten unter Rückfluss gekocht, im Vakuum eingeengt und an einer Kieselgelsäule zunächst mit Heptan-Aceton 1:1, dann mit Methanol-Aceton 1:1 chromatografisch gereinigt. Man erhält 2,6 g amorphe, beigefarbene Verbindung.

DC Kieselgelplatte; Laufmittel: Chloroform-Methylethylketon-Methanol-Eisessig-Wasser: 7,5/2,5/3,5/0,5/0,9

λmax. 353 nm, ε' = 8,7 $cm^2$ $\mu mol^{-1}$

Beispiel 5
2,5-Bis-(3,5-dimethoxy-4-hydroxyphenyl)-1,3-oxazol

a) 3,5-Dimethoxy-4-hydroxy-benzaldehydcyanhydrin

10 g (0,055 Mol) 3,5-Dimethoxy-4-hydroxy-benzaldehyd werden mit 10 g (0,1 Mol) Natriumhydrogensulfit in 100 ml Wasser unter Rühren erwärmt, wobei Lösung erfolgt. Danach tropft man unter Rühren 2,7 g (0,055 Mol) Natriumcyanid in 13 ml Wasser innerhalb 30 min zu, rührt 30 min nach und extrahiert das gebildete Cyanhydrin durch Ausschütteln mit 3 × 50 ml Ether. Nach Trocknen über Natriumsulfat wird die Lösung direkt für Stufe b) verwendet.

DC Kieselgel; Laufmittel: Chloroform-Methanol 5:1, RF-Wert: 0,88

Laufmittel: Chloroform-Tetrahydrofuran 1:1, RF-Wert: 0,8

b) Die Etherlösung aus 5a) wird mit 2,2 g (0,014 Mol) 3,5-Dimethoxy-4-hydroxy-benzaldehyd versetzt und unter Kühlung Chlorwasserstoffgas bis zur Sättigung eingeleitet. Beim Stehen kristallisieren 1,06 g (= 25% Ausbeute) der Titelverbindung, die man durch Aufnehmen in Wasser und Ausschütteln mit Dichlormethan rein erhält. Die farblosen Kristalle schmelzen bei 260 °C (Zers.).

λmax. 583 nm, ε' = 29,2 $cm^2$ $\mu mol^{-1}$

Beispiel 6
2-(3,5-Dimethoxy-4-hydroxyphenyl)-4-(N,N-bis-methoxymethylaminophenyl)-5-methyl-1,3-oxazol

5 g (0,0153 Mol) der Verbindung des Beispiels 3 (freie Base) werden in 300 ml Methanol gelöst, 20 ml 37%ige Formaldehydlösung zugegeben, 16 Stunden bei Raumtemperatur stehen gelassen und eingeengt. Der Rückstand wird mit 200 ml Ether angerieben. Man erhält 3 g (60,2% Ausbeute) hellgraue, amorphe Titelverbindung.

DC: Kieselgel; Laufmittel: Choroform-Methanol 19:1

RF-Wert: 0,58

λmax. 536 nm, ε' = 33,5 $cm^2$ $\mu mol^{-1}$

Beispiel 7
2-(3,5-Dimethoxy-4-hydroxyphenyl)-4-(4-dimethylaminophenyl)-5-methyl-1,3-oxazol-hydrochlorid

Man löst 3,2 g (0,0084 Mol) der Verbindung des Beispiels 3 (freie Base) in 110 ml Methanol/Wasser 10/1, gibt 1,9 ml 37%ige Formaldehydlösung, 1 ml konz. Chlorwasserstoffsäure sowie 0,5 g Platinoxid zu und hydriert vier Stunden bei 5–8 °C unter Normaldruck. Nach Abfiltrieren des Katalysators engt man auf 50 ml ein und erhält beim Kühlen mit Eis 2 g (51% Ausbeute) farblose Kristalle der Titelverbindung. Fp. > 250 °C (Zers.).

λmax. 510 nm, ε' = 30 $cm^2$ $\mu mol^{-1}$

7.1. 2-(3,5-Dimethoxy-4-hydroxyphenyl)-4-(3-methoxy-4-dimethyl-aminophenyl)-5-methyl-1,3-oxazol-hydrochlorid

In analoger Weise wie in Beispiel 7 beschrieben erhält man aus dem Hydrochlorid der Base Beispiel 3.1 mit Formaldehydlösung und Platinoxid als Katalysator 82% der Titelverbindung, farblose Kristalle, Fp. 230–235 °C (Zers.).

λmax. 499 nm, ε' = 29,2 $cm^2$ $\mu mol^{-1}$

Beispiel 8
2-(3,5-Dimethoxy-4-hydroxyphenyl)-4-[4-(2,3-dihydroxy-propyl)-aminophenyl]-5-methyl-1,3-oxazol

15,9 g (0,049 Mol) der Verbindung des Beispiels 3 (freie Base) werden in 320 ml Ethanol suspendiert und nach Zugabe von 19,1 ml (0,29 Mol) 2,3-Epoxy-propanol 5 Stunden unter Rückfluss gekocht, danach im Vakuum eingeengt und das Rohprodukt durch Chromatografie an Kieselgel gereinigt. Laufmittel: Chloroform-Methanol 12:1. Die entsprechenden Fraktionen ergeben 3,32 g (26,5% Ausbeute), schwach rosagefärbte Titelverbindung, Fp. 165–167 °C (Zers.).

λmax. 491, ε' = 21,6 $cm^2$ $\mu mol^{-2}$

8.1. 2-(3,5-Dimethoxy-4-hydroxyphenyl)-4-[4-bis-(2,3-dihydroxypropyl)aminophenyl]-5-methyl-1,3-oxazol

Durch Umsetzung von 3,8 g (0,01 Mol) 2-(3,5-Dimethoxy-4-hydroxyphenyl)-4-(4-aminophenyl)-5-methyl-oxazol und 1,41 ml (0,029 Mol) 2,3-Epoxypropanol durch 5stündiges Kochen unter Rückfluss erhält man neben der Monoverbindung Beispiel 8 auch die gewünschte Bisverbindung. Die Reinigung erfolgt durch Säulenchromatografie an Kieselgel 60. Säule Ø 4,5 cm, Füllhöhe 70 cm, Laufmittel: Methylenchlorid:Methanol 8:1. Die entsprechenden Fraktionen geben 2,1 g = 40% der Titelverbindung. Farblose Kristalle, Fp. 187 °C–189 °C (Zers.).

DC-Fertigplatte Kieselgel 60 F 254,
Laufmittel: Methylenchlorid/Methanol 8:1,
RF-Wert: 0,31
λmax. 510 nm, ε' = 23,6 $cm^2$ $\mu mol^{-1}$ (bei pH 6)
λmax. 505 nm, ε' = 33,3 $cm^2$ $\mu mol^{-1}$ (bei pH 8)

8.2. 2-(3,5-Di-tert.butyl-4-hydroxyphenyl)-4-(4-bis-2,3-dihydroxypropyl)aminophenyl]-5-methyl-1,3-oxazol aus 3,3 und 2,3-Epoxypropanol analog 8a)

Titelverbindung: gelbliches Öl

DC-Kieselgelplatte 60, Laufmittel: $CHCl_2$:$CHCl_3$ 8:1

RF-Wert: 0,31

λmax 552 nm, ε' = 30,3 $cm^2$ $\mu mol^{-1}$

Beispiel 9
2-(3,5-Dimethoxy-4-hydroxyphenyl)-4-[N-3-chlor-2-hydroxypropyl]-(aminophenyl)-5-methyl-1,3-oxazol

3,26 g (0,01 Mol) der Verbindung des Beispiels 3 (freie Base) werden in 65 ml Ethanol mit 1,12 ml (0,14 Mol) Epichlorhydrin sechs Stunden unter Rückfluss erhitzt, danach im Vakuum eingeengt, der Rückstand mit 25 ml Wasser und danach mit Ether angerührt. Man erhält 1,6 g (38,2% Ausbeute), farblose Kristalle der Titelverbindung. Fp. 120–123 °C (Zers.).

λmax. 554 nm, ε' = 37,2 $cm^2$ $\mu mol^{-2}$

Beispiel 10
2-(3,5-Dimethoxy-4-hydroxyphenyl)-4-[N-3-morpholino-2-hydroxypropyl]-(aminophenyl)-5-methyl-1,3-oxazol

4,2 g (0,01 Mol) der in Beispiel 9 aufgeführten Verbindung werden mit 50 ml Morpholin 1,5 Stunden unter Rückfluss erhitzt. Danach engt man im Vakuum ein, rührt den Rückstand mit Aceton an, filtriert das entstehende Morpholinhydrochlorid ab, wäscht dieses mit Aceton und engt das Filtrat abermals ein. Der Rückstand, 8 g bräunliches Öl, wird danach an Kieselgel 60 mit Isopropanol/n-Butylacetat/Wasser (5/3/2) chromatografisch gereinigt. Man erhält 1,7 g (36,4% Ausbeute) der Titelverbindung, honigfarbenes Öl.

DC: Kieselgelplatte: Laufmittel wie bei Säulentrennung.

RF-Wert: 0,51

λmax. 551 nm, ε' = 10 $cm^2$ $\mu mol^{-1}$

Beispiel 11
⟨⟨3-⟨4-[2-(3,5-Dimethoxy-4-hydroxyphenyl)-5-methyl-4-1,3-oxazolyl]-phenylamino⟩-2-hydroxypropyl⟩⟩phosphonsäure

a) 2,3-Epoxypropanphosphonsäurediethylester

60 g (0,44 Mol) Epibromhydrin werden mit 66,5 g = 68,6 ml (0,4 Mol) Triethylphosphat gemischt und 3 Stunden auf 110 °C erwärmt. Das entstehende Ethylbromid wird am absteigenden Kühler abdestilliert und das erhaltene Rohprodukt anschliessend fraktioniert destilliert. Man erhält 39,8 g = 51,2% Ester, Kp. $_{0,7}$: 93–95 °C (GC 96,5%)

b) ⟨⟨3-⟨4-[2-(3,5-Dimethoxy-4-hydroxyphenyl)-5-methyl-4-(1,3)-oxazolyl]phenylamino⟩-2-hydroxypropyl⟩⟩phosphonsäurediethylester

3,26 g (0,01 Mol) der Verbindung des Beispiels 3 (freie Base) werden in 100 ml Ethanol suspendiert, 11,65 g (0,06 Mol) Epoxidverbindung der Stufe 11a) zugegeben und 7 Stunden unter Rückfluss erhitzt. Nach Abdampfen des Lösungsmittels wird das resultierende hellbraune Öl an Kieselgel 60 chromatografisch gereinigt. Säule: Füllhöhe 73 cm, Laufmittel: Chloroform:Methanol (19:1). Die Aufarbeitung der entsprechenden Fraktion ergibt 3,5 g = 67,3% schwach gelb gefärbte Titelverbindung. (Fp. 188 °C)

DC-Fertigplatte: Kieselgel 60 F 254, Laufmittel: Chloroform-Methanol (8:1), RF-Wert 0,313

λmax 517 nm, ε' = 10,5 $cm^2$ $\mu mol^{-2}$

c) ⟨⟨3-⟨4-[2-(3,5-Dimethoxy-4-hydroxyphenyl)-5-methyl-4-1,3-oxazolyl]-phenylamino⟩-2-hydroxypropyl⟩⟩phosphonsäurehydrochlorid

2,3 g (0,044 Mol) Phosphonsäureester, Beispiel 11b) werden mit 25 ml 6 N HCl 3 Stunden unter Rückfluss erhitzt und danach im Vakuum eingeengt. Das erhaltene Rohprodukt, 2,3 g halbkristallines Material, wird mit 15 ml Isopropanol angerührt, abgesaugt und getrocknet. Man erhält 1,76 g = 88,8% beigefarbene Kristalle der Titelverbindung.

Fp. 170/182 °C (Zers.).

λmax 583 nm, ε' = 10,5 $cm^2$ $\mu mol^{-1}$

Beispiel 12
⟨⟨3-⟨4-[2-(3,5-Dimethoxy-4-hydroxyphenyl)]-5-methyl-4-(1,3)-oxazolyl-phenylmethylamino⟩-2-hydroxypropyl⟩⟩phosphonsäure

a) Titelverbindung: Diethylester

1 g (1,9 mmol) der Verbindung Beispiel 11b) werden in 100 ml Methanol mit 0,1 g $PtO_2$ in Gegenwart von 0,5 ml Formalin und 0,2 ml konzentrierter Salzsäure 4 Stunden bei Raumtemperatur hydriert. Nach Absaugen des Katalysators wird das Filtrat im Vakuum eingeengt. Man erhält 1,1 g rötliches Öl, dieses wird an einer Kieselgel-60-Säule (Ø 4 cm, Füllhöhe 70 cm) mit Chloroform-Methanol (19:1) chromatografisch gereinigt. Man erhält nach Einengen der entsprechenden Fraktion 0,31 g = 30,2% amorphes, farbloses Pulver.

DC-Fertigplatte: Kieselgel 60 F 254, Laufmittel: Chloroform-Methanol (19:1), RF-Wert 0,34

λmax 589 nm, ε' = 67,3 $cm^2$ $\mu mol^{-1}$

b) 0,3 g (0,56 mmol) der Verbindung 12a) werden in 10 ml 6 N HCl 2 Stunden unter Rückfluss erhitzt, danach im Vakuum zum Trocknen eingeengt und der Rückstand mit Isopropanol angereichert. Man erhält 2,73 g = 57,1% der Titelverbindung gelbliches, amorphes Pulver.

DC-Fertigplatte: Kieselgel 60 F 254, Laufmittel: Chloroform-Methanol (19:1), RF-Wert 0,26

λmax 593 nm, ε' = 9,7 $cm^2$ $\mu mol^{-1}$

Beispiel 13
2-(3,5-Di-tert.butyl-4-hydroxyphenyl)-4-⟨4-[N,N-bis-(2,3-dihydroxypropyl)]aminophenyl⟩-5-methyl-1,3-oxazol

3,8 g (0,01 Mol) 2-(3,5-Di-tert.butyl-4-hydroxyphenyl)-4-(4-aminophenyl)-5-methyl-1,3-oxazol (Beispiel 3.3) werden mit 3,7 g = 3,3 ml (0,05 Mol) 2,3-Epoxy-1-propanol in 500 ml Ethanol 8 Stunden unter Rückfluss gekocht. Danach wird im Vakuum eingeengt und der Rückstand säulenchromatografisch gereinigt. Säule Ø 4,5 cm, Füllhöhe 70 cm, Kieselgel 60, Laufmittel: Methylenchlorid:Methanol (8:1). Durch Einengen der entsprechenden Fraktionen erhält man 2,4 g = 45,6% amorphes, leicht rötliches Pulver.

DC-Fertigplatte: Kieselgel 60 F 254, Laufmittel: Methylenchlorid-Chloroform (8:1), RF-Wert 0,31

λmax 395 nm, ε' = 22,2 $cm^2$ $\mu mol^{-1}$

Beispiel 14
N-⟨4-[2-(3,5-Di-tert.butyl-4-hydroxyphenyl)-5-methyl-4-(1,3-oxazolyl]-phenylamino⟩essigsäure

a) N-⟨4-[2-(3,5-Di-tert.butyl-4-hydroxyphenyl)-5-methyl-4-(1,3)-oxazolyl]phenylamino>essigsäurebenzylester

3,78 g (0,01 Mol) 2-(3,5-Di-tert.butyl-4-hydroxyphenyl)-4-(4-aminophenyl)-5-methyl-1,3-oxazol werden mit 2,52 g (0,011 Mol) Bromessigsäurebenzylester in 50 ml absolutes Dimethylformamid in Gegenwart von 2,36 g (0,011 Mol) 1,8-Bis-dimethylaminonaphthalin 2,5 Stunden unter Argon gerührt, danach im Vakuum eingeengt, Rückstand in 100 ml Methylenchlorid aufgenommen, mehrmals mit Wasser durchgeschüttelt, organische Phase über Natriumsulfat getrocknet und eingeengt. Man erhält 5,94 g rötliches, amorphes Rohprodukt, das säulenchromatografisch gereinigt wird. Säule Ø 4,5 cm, Füllhöhe 85 cm, Kieselgel 60, Laufmittel: Chloroform und 1% Essigester. Durch Einengen der entsprechenden Fraktion erhält man 3,11 g = 71,4% d.Th. der Titelverbindung amorphes, leicht rötliches Pulver.

DC-Fertigplatte: Kieselgel 60 F 254, Laufmittel: Chloroform und 1% Essigester, RF-Wert 0,29

b) 2,3 g (4,4 mmol) des Benzylesters werden in 100 ml Methanol gelöst und nach Zugabe von 0,2 g Palladium auf Aktivkohle (10%ig) 30 Minuten bei Raumtemperatur hydriert. Nach Absaugen des Katalysators engt man die Lösung im Vakuum ein und erhält 1,92 g = 98% hellrote, amorphe Titelverbindung.

DC-Fertigplatte: Kieselgel 60 F 254, Laufmittel: Isopropanol/n-Butylacetat/$H_2O$ (5/3/2), RF-Wert 0,53

λmax 510 nm, $\varepsilon' = 32{,}3\ cm^2\ \mu mol^{-1}$

Beispiel 15

2-(3,5-Dimethoxy-4-hydroxyphenyl)-4-(4-dimethylaminophenyl)-1,3-thiazol

a) 2-(3,5-Dimethoxy-4-benzyloxyphenyl)-4-(4-dimethylaminophenyl)-1,3-thiazol

3,7 g (0,012 Mol) 3,5-Dimethoxy-4-benzyloxyphenylthioamid und 2,94 g (0,012 Mol) 4-Dimethylamino-α-bromacetophenon werden in 120 ml abs. Ethanol drei Stunden unter Rückfluss erhitzt. Nach Abkühlen und Einstellen in ein Eisbad erhält man 5,21 g (97,3% Ausbeute) hellgelbe Kristalle der Titelverbindung. Fp. 205–207 °C (Zers.).

b) Das vorgenannte Produkt wird in 250 ml Methanol gelöst und in die siedende Lösung zwei Stunden Chlorwasserstoffgas eingeleitet. Nach Einengen im Vakuum wird der Rückstand in 150 ml Wasser gelöst und das Thiazol durch Zugabe von Ammoniak in Freiheit gesetzt. Nach Aufnehmen mit Dichlormethan und Aufarbeitung erhält man nach Umkristallisieren aus Ethanol 3,1 g (65,7% Ausbeute) der Titelverbindung, schwach gelbliche Kristalle, Fp. 219–221 °C (Zers.).

λmax 541 nm, $\varepsilon' = 12{,}2\ cm^2\ \mu mol^{-1}$

Beispiel 16

2-(3,5-Dimethoxy-4-hydroxyphenyl)-4,5-bis-(4-dimethylaminophenyl)-1,3-thiazol-hydrochlorid

a) 2-(3,5-Dimethoxy-4-benzyloxyphenyl)-4,5-bis-(4-dimethylaminophenyl)-1,3-thiazol

5,68 g (0,01 Mol) der Titelverbindung Beispiel 4c werden in 60 ml abs. Chloroform gelöst und unter Zugabe von 3,3 g (0,015 Mol) Phosphorpentasulfid zwei Stunden unter Rühren am Rückfluss erhitzt. Danach giesst man auf ein Gemisch von Ammoniak und Eis, trennt die organische Phase ab und schüttelt diese mehrfach mit Wasser durch. Nach Trocknen und Einengen erhält man 5,8 g Titelverbindung 16a), braunes Öl.

DC-Kieselgelplatte 60, Laufmittel: Essigester RF-Wert 0,88

b) Das vorgenannte Produkt wird mit 30 ml 6 N HCl 30 Stunden unter Rückfluss erhitzt, nach Zugabe von Ammoniak 3 × mit 30 ml Dichlormethan extrahiert, aufgearbeitet und säulenchromatografisch an Kieselgel gereinigt. Laufmittel: Methanol/Chloroform (6/1). Die entsprechenden Fraktionen enthalten 2,2 g (46,2% Ausbeute) der Titelverbindung, gelbliche Kristalle. Fp. 218 °C (Zers.).

λmax 552 nm, $\varepsilon' = 10{,}8\ cm^2\ \mu mol^{-1}$

Beispiel 17

Übersicht über die optischen Eigenschaften der Substanzen der allgemeinen Formel 1

Zur Ermittlung des molaren Extinktionskoeffizienten wird wie folgt vorgegangen: $2 \times 10^{-2}$ mol Indikator der allgemeinen Formel I werden in 100 ml 0,1 m Salzsäure gelöst. Löst sich eine Substanz nicht quantitativ auf, wird in einem Gemisch Salzsäure/Methanol 9:1 gelöst. Von dieser Lösung werden 0,1 ml mit 10 ml 0,1 m Phosphatpuffer pH 6,0 verdünnt. Von der so erhaltenen Indikatorlösung werden 10 µl in ein Gemisch, bestehend aus 10 µl verdünntes $H_2O_2$ (100 µl 30%iges $H_2O_2$ werden auf 100 ml mit Wasser verdünnt), 10 µl Peroxidaselösung (600 U Peroxidase werden in 1 ml Wasser gelöst) und 10 ml 0,1 m Phosphatpuffer pH 6,0 pipettiert. Der Indikator wird oxidiert, die Lösung verfärbt sich. Von der gefärbten Lösung wird nach 60 Sekunden ein Spektrum geschrieben und aus den Extinktionswerten die molaren Extinktionskoeffizienten berechnet (fällt der gebildete Farbstoff aus, so wird ein Gemisch aus Puffer, Aceton oder Methanol 9:1 verwendet).

Nach dem gleichen Verfahren lassen sich aus Proben die $H_2O_2$-Konzentrationen ermitteln bzw. die Konzentrationen von Substraten, aus denen durch eine vorgeschaltete enzymatische Reaktion als ein Reaktionsprodukt $H_2O_2$ entsteht.

Beispiel 18

Testsystem zum Nachweis von Harnsäure in wässrigen Lösungen

Auf eine mit Gelatine vorbeschichtete Polyesterfolie wird mit einer Nassfilmdicke von 300 µm eine Gelatinematrix der unten aufgeführten Zusammensetzung gegossen und anschliessend getrocknet. In 47,5 ml Tris-Phosphat-Puffer 0,5 m, pH 7,2, werden 8,4 g Gelatine, 0,25 g Tween® 20, 0,5 kU Uricase, 5 kU Peroxidase, 100 mg Indikator-Substanz aus Beispiel 3 (2-(3,5-Dimethoxy-4-hydroxyphenyl)-4-(4-aminophenyl)-5-methyl-1,3-oxazol-hydrochlorid) eingearbeitet. Der so hergestellte Reagenzfilm wird zu einem Testsystem entsprechend Abb. 1 weiterverarbeitet.

Auf die Dosierzone werden 35 µl Harnsäurelösung aufgebracht. Durch Andrücken der Reagenzzone und des Gewebes auf die Transportzone

wird die Reaktion gestartet. Nach 2 Minuten wird in einem Remissionsphotometer gemessen (das Gewebe hat die Funktion, die Unebenheiten des Glasfaservlieses zu egalisieren).

Die mit dem beschriebenen System erhaltenen Eichkurven werden in der folgenden Tabelle wiedergegeben:

| Harnsäurekonzentration | % Remission |
|---|---|
| 3 mg/dl | 54,9 |
| 5 mg/dl | 48,8 |
| 7 mg/dl | 43,2 |
| 9 mg/dl | 38,7 |
| 11 mg/dl | 33,6 |
| 14 mg/dl | 30,9 |

Beispiel 19

Testsystem zum Nachweis von Creatinin im Serum

Ein saugfähiger Träger (Schablonenpapier der Fa. Schöller und Hösch, Flächengewicht 12 g/$m^2$, Saugfähigkeit 50 ml/$m^2$) wird mit einer Lösung von 200 kU Peroxidase und 1,2 g Kollagenhydrolysat gelöst in 100 ml Phosphatpuffer 0,1 m, pH 8,0 imprägniert und getrocknet. In einem zweiten Imprägniervorgang wird das vorimprägnierte Papier mit einer Lösung, bestehend aus 2 mmol Indikator-Substanz aus Beispiel 8a) (2-(3,5-Dimethoxy-4-hydroxyphenyl)-4[4-bis-(2,3-dihydroxypropyl)aminophenyl]-5-methyl-1,3-oxazol) in 100 ml Methanol nachimprägniert und getrocknet. Man erhält das Reagenzpapier a).

Zur Herstellung des Reagenzpapiers b) wird der o.g. saugfähige Träger mit einer Lösung von 5 kU Sarkosinoxidase, 30 kU Creatininamidohydrolase und 40 kU Creatininamidinohydrolase, 0,5 g Triton®X 100 in 100 ml 0,1 m Phosphatpuffer pH 8,0 imprägniert und getrocknet.

Beide Papiere werden entsprechend der Abb. 2 in ein Testsystem eingearbeitet.

Zum Nachweis von Creatinin im Serum pipettiert man 30 µl Serum auf die Dosierzone. Durch das Andrücken des Enzym- und Indikatorpapiers auf die Transportzone wird die Reaktion gestartet. Die entsprechende Farbe wird nach einer Minute remissionsphotometrisch vermessen. Die Auswertung erfolgt über eine Eichkurve.

In der folgenden Tabelle werden die Werte für eine Eichkurve für Creatinin im Serum wiedergegeben:

| Creatininkonzentration | % Remission |
|---|---|
| 0.1 mg/dl | 68,1 |
| 0.5 mg/dl | 59,9 |
| 1.5 mg/dl | 48,2 |
| 5,0 mg/dl | 35,0 |
| 10,0 mg/dl | 30,7 |

Beispiel 20

Testsystem zum Nachweis von Harnsäure in Blut

Aus den unten aufgeführten Bestandteilen wird eine Beschichtungsmasse hergestellt und mit einer Nassfilmdicke von 200 µm auf einer transparenten Folie aufgerakelt und getrocknet. 18 g einer Kunststoffdispersion eines Mischpolymeren aus Vinylacetat und Vinylpropionat, 1,5 g Alginat, 68 ml 0,5 m Tris-Citrat-Puffer pH 7,5, 0,7 g Indikator-Substanz aus Beispiel 3, 2 kU Uricase, 100 kU Peroxidase, 0,5 g Triton®X 100 und 12 g Diatomeenerde werden homogen verrührt.

Auf die so hergestellte Schicht wird eine zweite Schicht als optischer, weisser Hintergrund der unten aufgeführten Zusammensetzung mit einer Schichtdicke von 200 µm gerakelt und getrocknet. 52 ml 0,1 m Tris-Citrat-Puffer pH 7,0, 5,5 g Titandioxid, 2,7 g Diatomeenerde, 0,4 g Alginat, 1,4 g einer Kunststoffdispersion eines Mischpolymerenzusatzes aus Vinylacetat und Vinylpropionat, 0,2 g Triton®X 100.

Der so hergestellte Testfilm wird zu Testen gemäss Abb. 3 verarbeitet.

Zum Nachweis von Harnsäure aus Blut werden 30 µl Blut auf die Dosierzone aufgetragen, die Reagenzklappe wird nach einer Minute angedrückt, und nach weiteren zwei Minuten wird mit einem Remissionsphotometer die gebildete Farbe vermessen und aus einer vorher ermittelten Eichkurve die Harnsäurewerte ermittelt.

In der folgenden Tabelle werden die Werte für die Eichkurve wiedergegeben.

| Harnsäurekonzentration | % Remission |
|---|---|
| 4,6 mg/dl | 58,0 |
| 6,0 mg/dl | 50,6 |
| 7,9 mg/dl | 43,1 |
| 9,0 mg/dl | 40,5 |
| 10,6 mg/dl | 36,1 |
| 13,5 mg/dl | 31,8 |
| 15,6 mg/dl | 28,7 |

Beispiel 21

Testsystem zum Nachweis von GPT im Blut

Je ein saugfähiger Träger (Schablonenpapier der Firma Schöller und Hösch, Flächengewicht 12 g/$m^2$, Saugfähigkeit 50 ml/$m^2$) wird mit den unten aufgeführten Lösungen 1 und 2 imprägniert und getrocknet. Lösung 1: In einem Liter eines 0,2 m Puffers aus Kalilauge und 2-(N-Morpholino)-ethansulfonsäure von pH 6,7 werden gelöst: 0,03 mol α-Ketoglutarat, 0,8 mol Alanin, 0,01 mol Magnesiumchlorid, 0,0001 mol Ascorbinsäure, 0,009 mol Substanz aus Beispiel 7 (2-(3,5-Dimethoxy-4-hydroxyphenyl)-4-(dimethylaminophenyl)-5-methyl-1,3-oxazol-hydrochlorid) und 5 g Octylpyranosid. Man erhält das Reagenzpapier a).

Lösung 2: In einem Liter des o.g. Puffers werden gelöst: 0,003 mol Thiaminpyrophosphat, 500 kU Pyruvatoxidase, 500 kU Peroxidase, 100 kU Ascorbatoxidase. Man erhält das Reagenzpapier b).

Diese Reagenzpapiere werden zu einem Testsystem gemäss Abb. 2 verarbeitet.

Zur Bestimmung der Enzymaktivität werden 30 µl Blut auf die Dosierzone pipettiert, nach einer

Minute die Deckfolie und die Reaktionspapiere angedrückt und die Farbentwicklung nach der Zeit mit einem Remissionsphotometer verfolgt. Die Auswertung erfolgt über eine Zweipunktmessung aus einer Bezugskurve. Die Bezugskurve wird erstellt, indem man Verdünnungsreihen mit Enzymaktivitäten von 10–1000 U/l herstellt und im Remissionsphotometer die Remissionswerte über Fixtimemessungen erarbeitet.

Beispiel 22
Verfahren zum Nachweis von Glucosekonzentrationen im Blut zur Diagnose einer Hypoglycämie

Eine Rohfilmmasse wird wie folgt hergestellt:

10 g einer 1,7%igen Alginatanquellung in einem 0,5 m Phosphatpuffer pH 5,0, 15 g wässrige Kunststoffdispersion eines Copolymeren aus Vinylacetat und Vinylpropionat, 5 g einer 15%igen wässrigen Lösung von 4-Dodecylbenzolsulfonat, 25 kU Glucoseoxidase, 200 kU Peroxidase, 270 mg Substanz aus Beispiel 3), 10 g Diatomeenerde, 0,4 ml Hexanol werden zu einem homogenen Brei verrührt und mit einer Nassfilmdicke von 150 µm auf ein multifiles Gewebe (2 F/964 der Firma Schweizer Seidengaze Fabrik) aufgerakelt und anschliessend getrocknet.

Dieser Film wird zu einem Testsystem gemäss Abb. 4 verarbeitet. Zur Bestimmung der Glucose pipettiert man 30 µl Blut auf die Dosierzone, drückt die Deckfolie und den Reagenzfilm auf die Transportzone und vermisst die entstandene Reaktionsfarbe mit einem Remissionsphotometer. Die Glucosekonzentrationen werden anhand einer Eichkurve ermittelt, die das unten angegebene Aussehen hat.

| mg Glucose/dl | % Remission |
|---|---|
| 20 | 37,2 |
| 40 | 22,6 |
| 60 | 18,6 |
| 80 | 12,8 |

Bezugszeichenliste

1. Reagenzzonen-Träger (transparent)
2. Reagenzzone
3. Gewebe
4. Transportzone aus Glasfasern
5. Abtrennzone aus Glasfasern
6. Fixiergewebe
7. Deckfolie (transparent)
8. Reagenzpapier a)
9. Reagenzpapier b)
10. Optisch weisser Hintergrund (porös)
11. Multifiles Gewebe
12. Trägerfolie
13. Klebestelle

**Patentansprüche**

1. Verbindungen der Formel I

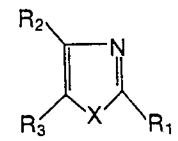

(I),

in der

X Sauerstoff oder Schwefel ist,

$R_1$ Julolidin oder Tetrahydrochinolin, das am Stickstoffatom eine Alkylgruppe mit 1–6 Kohlenstoffatomen tragen kann, die wiederum durch einen Sulfonsäure-, Phosphonsäure- oder Carbonsäurerest substituiert sein kann, oder die Gruppe

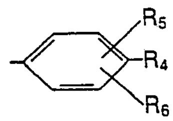

in der

$R_4$ eine Hydroxy-, eine mono- oder dialkylierte Aminogruppe, wobei die Alkylgruppen 1–6 Kohlenstoffatome aufweisen und ein- oder mehrfach durch Halogen, Hydroxy, Methoxy, Carboxyl, einen Sulfonsäure- oder Phosphonsäurerest, der durch Methanol oder Ethanol verestert sein kann, oder einen Morpholinorest substituiert sein können,

$R_5$ und $R_6$, die gleich oder verschieden sein können, Wasserstoff, $C_1$–$C_6$-Alkyl, das ein- oder mehrfach durch Halogen, Hydroxy, Methoxy, Carboxyl, einen Sulfonsäure- oder Phosphonsäurerest, der durch Methanol oder Ethanol verestert sein kann, oder einen Morpholinorest substituiert sein kann, oder $C_1$–$C_6$-Alkoxy, das durch eine Carboxylgruppe substituiert sein kann, bedeuten,

$R_2$ Wasserstoff, einen Alkylrest mit 1–6 Kohlenstoffatomen, der ein- oder mehrfach durch Halogen, Hydroxy, Methoxy, Carboxyl, einen Sulfonsäure- oder Phosphonsäure-Rest, der durch Methanol oder Ethanol verestert sein kann, oder einen Morpholinorest substituiert sein kann, sowie Julolidin, Tetrahydrochinolin, das am Stickstoffatom eine Alkylgruppe mit 1–6 Kohlenstoffatomen tragen kann, die durch einen Carboxyl-, Phosphonsäure- oder Sulfonsäurerest substituiert sein kann, oder die Gruppe

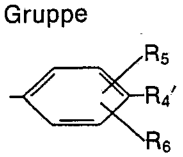

in der

$R_5$ und $R_6$ die bei $R_1$ angegebene Bedeutung haben und

$R_4'$ eine Hydroxy-, eine Amino- oder eine mono- oder dialkylierte Aminogruppe darstellt, wobei die Alkylgruppen 1–6 Kohlenstoffatome aufweisen und ein- oder mehrfach durch Halogen, Hydroxy, $C_1$–$C_6$-Alkoxy, Carboxyl, einen Sulfonsäure- oder Phosphonsäurerest, der durch Methanol oder Ethanol verestert sein kann, oder einen Morpholinorest substituiert sein können,

bedeuten und

$R_3$ die gleiche Bedeutung wie $R_2$ hat oder Cycloalkyl mit 3–7 Kohlenstoffatomen, Phenyl, Pyridyl oder einen durch Di-$C_1$–$C_6$-alkylamino oder Phenyl substituierten $C_1$–$C_6$-Alkylrest darstellt, sowie deren Salze.

2. Verfahren zur Herstellung von Verbindungen gemäss Anspruch 1, dadurch gekennzeichnet, dass man in an sich bekannter Weise

a) eine Verbindung der Formel II

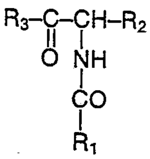

(II)

in der

$R_1$, $R_2$ und $R_3$ die in Anspruch 1 angegebene Bedeutung haben, mit einer Lewis-Säure oder Pentasulfid umsetzt, oder

b) für den Fall, dass X Sauerstoff bedeutet, eine Verbindung der Formel III

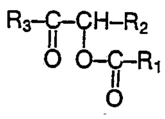

(III)

in der

$R_1$, $R_2$ und $R_3$ die in Anspruch 1 angegebene Bedeutung haben, mit Ammoniumacetat in Eisessig umsetzt, oder

c) für den Fall, dass X Schwefel bedeutet, ein Thioamid der Formel IV

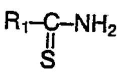

(IV)

in der

$R_1$ die in Anspruch 1 angegebene Bedeutung hat,

mit einem α-Halogenketon der Formel V

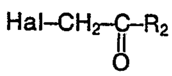

(V)

in der

$R_2$ die in Anspruch 1 angegebene Bedeutung hat und

Hal Fluor, Chlor oder Brom darstellt, umsetzt, wobei in den Substituenten $R_1$, $R_2$ und $R_3$ gegebenenfalls vorhandene phenolische Hydroxygruppen sowie Carboxylgruppen durch übliche Sauerstoffschutzgruppen geschützt sein können, die nach Durchführen der Umsetzung wieder abgespalten werden, und anschliessend gegebenenfalls erhaltene Salze der Verbindungen der Formel I in die entsprechenden Basen oder gegebenenfalls erhaltene Basen der Formel I in entsprechende Salze überführt.

3. Verwendung von Verbindungen gemäss Anspruch 1 als Redoxindikatoren.

4. Redoxindikatoren gemäss Anspruch 1 zum Nachweis von Wasserstoffperoxid oder peroxidatisch wirksamen Substanzen.

5. Reagenz zum Nachweis von Wasserstoffperoxid oder peroxidatisch wirksamen Substanzen, bestehend aus einer Verbindung der Formel I und üblichen Wirk- und Hilfsstoffen.

6. Reagenz gemäss Anspruch 5, dadurch gekennzeichnet, dass es in Form von Tabletten, Lyophylisaten, imprägnierten Reagenzträgern oder Reagenzfilmen Verwendung findet.

**Claims**

1. Compounds of the formula I

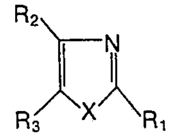

(I)

in which X is an oxygen or sulphur, $R_1$ signifies julolidine or tetrahydroquinoline which can carry on the nitrogen atom and alkyl group with 1–6 carbon atoms which, in turn, can be substituted by a sulphonic acid, phosphonic acid or carboxylic acid residue, or the group

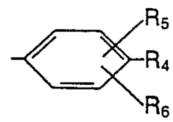

in which $R_4$ signifies a hydroxyl, a mono- or dialkylated amino group, whereby the alkyl groups have 1–6 carbon atoms and can be substituted one or more times by halogen, hydroxyl, methoxy, carboxyl, a sulphonic acid or phosphonic acid residue, which can be esterified by methanol or ethanol, or a morpholino radical, $R_5$ and $R_6$, which can be the same or different, signify hydrogen, $C_1$–$C_6$-alkyl, which can be substituted one or more times by halogen, hydroxyl, methoxy, carboxyl, a sulphonic acid or phosphonic acid radical, which can be esterified by methanol or ethanol, or a morpholino radical, or $C_1$–$C_6$-alkoxy, which can be substituted by a carboxyl group, $R_2$ signifies hydrogen, an alkyl radical with 1–6 carbon atoms, which can be substituted one or more times by halogen, hydroxyl, methoxy, carboxyl, a sulphonic acid or phosphonic acid radical, which can be esterified by methanol or ethanol, or a morpholino radical, as well as julolidine, tetrahydroquinoline which can carry on the nitrogen atom an alkyl group with 1–6 carbon atoms which can be substituted by a carboxyl, phosphonic acid or sulphonic acid radical, or the group:

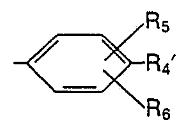

in which $R_5$ and $R_6$ have the meanings given in the case of $R_1$ and $R_4'$ signifies a hydroxyl, an amino or a mono- or dialkylated amino group, whereby the alkyl groups have 1–6 carbon atoms and can be substituted one or more times by halogen, hydroxyl, $C_1$–$C_6$-alkoxy, carboxyl, a sulphonic acid or phosphonic acid radical, which can be esterified by methanol or ethanol, or a morpholino radical and $R_3$ has the same meaning as $R_2$ or represents cycloalkyl with 3–7 carbon atoms, phenyl, pyridyl or $C_1$–$C_6$-alkyl radical substituted by di-$C_1$–$C_6$-alkylamino or phenyl, as well as their salts.

2. Process for the preparation of compounds according to claim 1, characterised in that, in per se known manner, one

a) reacts a compound of the formula II

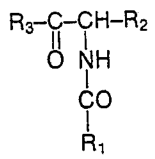
(II)

in which $R_1$, $R_2$ and $R_3$ have the meaning given in claim 1, with a Lewis acid or pentasulphide; or

b) for the case in which X signifies oxygen, reacts a compound of the formula III

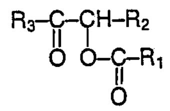
(III)

in which $R_1$, $R_2$ and $R_3$ have the meaning given in claim 1, with ammonium acetate in glacial acetic acid; or

c) for the case in which X signifies sulphur, reacts a thioamide of the formula IV

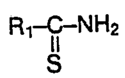
(IV)

in which $R_1$ has the meaning given in claim 1, with an α-halogenoketone of the formula V

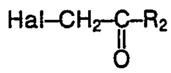
(V)

in which $R_2$ has the meaning given in claim 1 and Hal represents fluorine, chlorine or bromine; whereby phenolic hydroxyl groups possibly present in the substituents $R_1$, $R_2$ and $R_3$, as well as carboxyl groups, can be protected by usual oxygen protective groups, which, after carrying out the reaction, can again be split off, and subsequently salts of the compounds of formula I possibly obtained are converted into the corresponding bases or bases of formula I possibly obtained are converted into corresponding salts.

3. Use of compounds according to claim 1 as redox indicators.

4. Redox indicators according to claim 1 for the detection of hydrogen peroxide or of peroxidate-active substances.

5. Reagent for the detection of hydrogen peroxide or of peroxidate-active substances, consisting of a compound of formula I and usual active and adjuvant substances.

6. Reagent according to claim 5, characterised in that it finds use in the form of tablets, lyophilisates, impregnated reagent carriers or reagent films.

**Revendications**

1. Composé de formule I

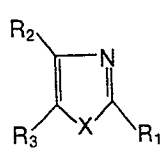
(I),

dans lequel

X représente l'oxygène ou le soufre,

$R_1$ représente la julolidine ou la tétrahydroquinoline, dont l'atome d'azote peut porter un groupe alkyle avec 1–6 atomes de carbone, qui à leur tour peuvent être substitués par un groupe acide sulfonique, un groupe acide phosphonique ou un groupe acide carboxylique, ou par le groupe

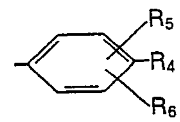

dans lequel

$R_4$ représente un groupe hydroxy, un groupe mono- ou dialkylamino, les groupes alkyle comprenant 1–6 atomes de carbone et pouvant être substitués une ou plusieur fois par un atome d'halogène, un groupe hydroxy, méthoxy, carboxyle, un groupe acide sulfonique ou un groupe acide phosphonique, qui peuvent être esterifiés par le méthanol ou l'éthanol,

$R_5$ et $R_6$, qui peuvent être identiques ou différents, représentent un atome d'hydrogène, un groupe alkyle en $C_1$–$C_6$, qui peut être substitué une ou plusieurs fois par un atome d'halogène, un groupe hydroxy, méthoxy, carboxyle, un groupe acide sulfonique ou un groupe acide phosphonique, qui peuvent être esterifiés par le méthanol ou l'éthanol, ou un groupe morpholino, ou un groupe alcoxy en $C_1$–$C_6$, qui peut être substitué par un groupe carboxyle,

$R_2$ représente un atome d'hydrogène, un groupe alkyle comprenant 1–6 atomes de carbone, qui peut être substitué une ou plusieurs fois par un atome d'halogène, un groupe hydroxy, méthoxy, carboxyle, un groupe acide sulfonique ou un groupe acide phosphonique, qui peuvent être esterifiés par le méthanol ou l'éthanol, ou un groupe morpholino, ainsi que la julolidine, la tétrahydroquinoline, dont l'atome d'azote peut porter un groupe alkyle avec 1–6 atomes de carbone, qui peut être substitué par un groupe acide sulfonique, un groupe acide phosphonique ou un groupe acide carboxylique, ou par le groupe

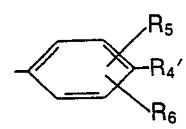

dans lequel

$R_5$ et $R_6$ ont la même signification que dans $R_1$, et

$R_4'$ représente un groupe hydroxy, un groupe amino, un groupe mono- ou dialkylamino, les groupes alkyle comprenant 1–6 atomes de carbone, et pouvant être substitués par un atome d'halogène, un groupe hydroxy, un groupe alcoxy en $C_1$–$C_6$, un groupe carboxyle, un groupe acide sulfonique ou un groupe acide phosphonique, qui peuvent être esterifiés par le méthanol ou l'éthanol, ou un groupe morpholino,

et

$R_3$ a la même signification que $R_2$ ou il représente un groupe cycloalkyle comprenant 3–7 atomes de carbone, un groupe phényle, un groupe pyridyle ou un groupe alkyle en $C_1$–$C_6$ substitué par un groupe dialkylamino en $C_1$–$C_6$ ou un groupe phényle,
ainsi que leurs sels.

2. Procédé de préparation de composés selon la revendication 1, caractérisé en ce que, de manière connue en soi, on fait réagir

a) un composé de formule II

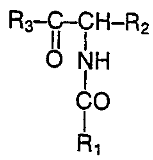
(II)

dans lequel
$R_1$, $R_2$ et $R_3$ ont la signification indiquée dans la revendication 1, avec un acide de Lewis ou un pentasulfure, ou

b) dans le cas où X représente l'oxygène, un composé de formule III

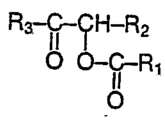
(III)

dans lequel
$R_1$, $R_2$ et $R_3$ ont la signification indiquée dans la revendication 1, avec de l'acétate d'ammonium dans l'acide acétique cristallisé, ou

c) dans le cas où X représente le soufre, un thioamide de formule IV

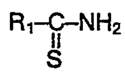
(IV)

dans lequel
$R_1$ a la signification indiquée dans la revendication 1, avec une α-halogènocétone de formule V

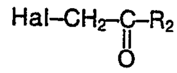
(V)

dans laquelle
$R_2$ a la signification indiquée dans la revendication 1, et
Hal représente le fluor, le chlore ou le brome, les groupes hydroxy phénoliques ainsi que les groupes carboxyle, éventuellement présents dans les substituants $R_1$, $R_2$ et $R_3$, pouvant être protégés par des groupes de protection usuels vis-à-vis de l'oxygène, ces groupes pouvant être séparés à nouveau après le déroulement de la réaction,
et qu'ensuite on transforme les sels éventuellement obtenus du composé de formule I en bases correspondantes, ou les bases éventuellement obtenues de formule I en sels correspondants.

3. Utilisation des composés de formule I comme indicateurs redox.

4. Indicateurs redox selon la revendication 1, destinés à déceler le peroxyde d'hydrogène ou les substances à action peroxydante.

5. Réactif pour déceler le peroxyde d'hydrogène ou les substances à action peroxydante, constitué par un composé de formule I et les matières actives et auxiliaires habituelles.

6. Réactif selon la revendication 5, caractérisé en ce qu'il est utilisé sous la forme de comprimés, lyophylisats, supports de réactifs imprégnés ou films de réactif.

Abb.1

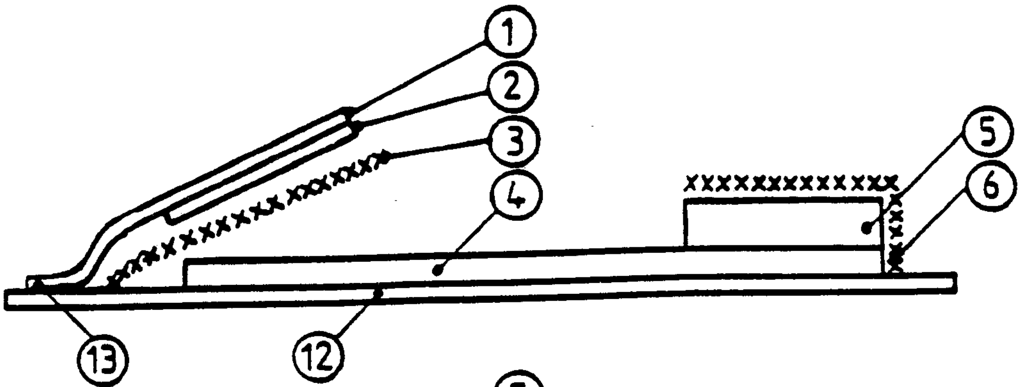

Abb.2

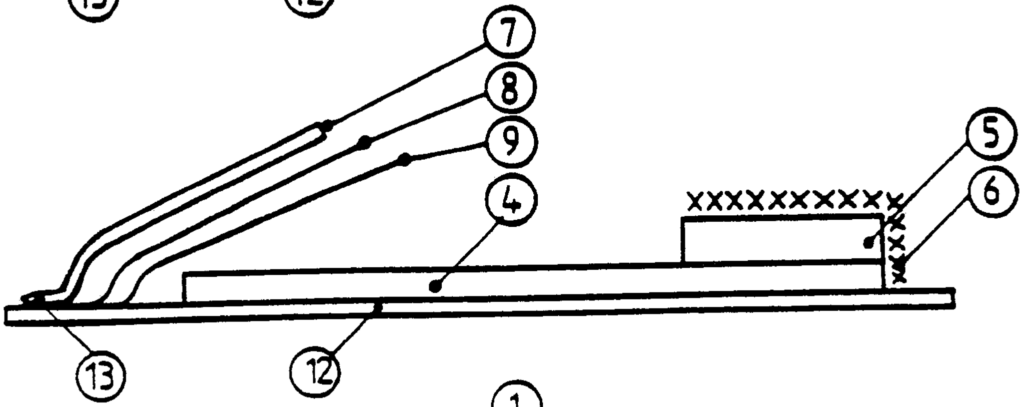

Abb. 3

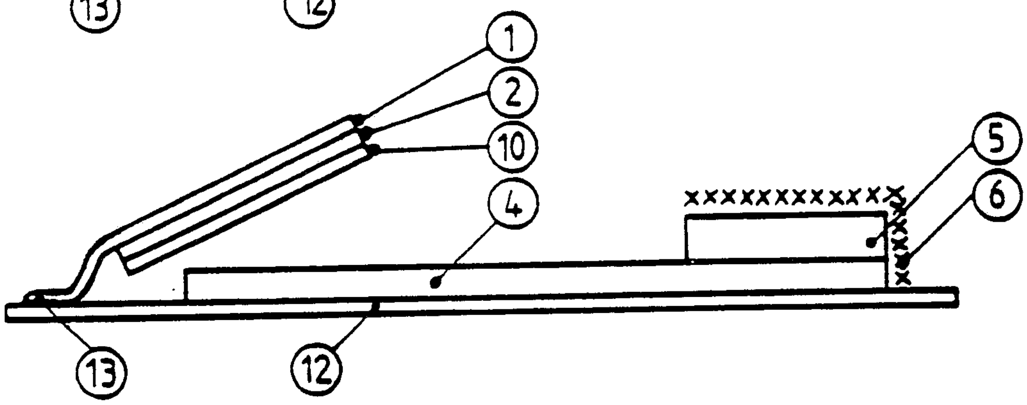

Abb.4